(19) Europäisches Patentamt
European Patent Office
Office européen des brevets

(11) Numéro de publication : **0 284 461 B1**

# (12) FASCICULE DE BREVET EUROPEEN

(45) Date de publication du fascicule du brevet : **12.06.91 Bulletin 91/24**

(51) Int. Cl.[5] : **C07C 237/22,** C07K 5/06, A61K 31/195, A61K 31/22, A61K 31/48

(21) Numéro de dépôt : **88400446.6**

(22) Date de dépôt : **26.02.88**

## (54) Nouveaux dérivés de l'acide glutamique, leurs sels, procédé de préparation, application à titre de médicaments et compositions les renfermant.

Le dossier contient des informations techniques présentées postérieurement au dépôt de la demande et ne figurant pas dans le présent fascicule.

(30) Priorité : **26.02.87 FR 8702547**

(43) Date de publication de la demande : **28.09.88 Bulletin 88/39**

(45) Mention de la délivrance du brevet : **12.06.91 Bulletin 91/24**

(84) Etats contractants désignés : **AT BE CH DE ES FR GB GR IT LI LU NL SE**

(56) Documents cités :
**EP-A- 0 025 842**
**FR-A- 2 566 410**

(73) Titulaire : **ROUSSEL-UCLAF**
**35, boulevard des Invalides**
**F-75007 Paris (FR)**

(72) Inventeur : **Agouridas, Constantin**
**67, Quai d'Orsay**
**F-75007 Paris (FR)**
Inventeur : **Fauveau, Patrick**
**40, Avenue Camille Desmoulins**
**F-93190 Livry-Gargan (FR)**
Inventeur : **Damais, Chantal**
**24-26, rue du Cotentin**
**F-75015 Paris (FR)**

(74) Mandataire : **Tonnellier, Marie-José et al**
**111, route de Noisy B.P. no 9**
**F-93230 Romainville (FR)**

**EP 0 284 461 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des dérivés de l'acide glutamique ainsi que leurs sels, le procédé de préparation, l'application à titre de médicaments de ces dérivés et les compositions les renfermant.

Des dérivés de l'acide diaminopimélique doués de propriétés antibactériennes ont été décrits dans la demande de brevet français n° 2 566 410.

L'invention a pour objet sous toutes leurs formes isomères ou sous formes de mélanges d'isomères des dérivés de l'acide glutamique ainsi que leurs sels d'addition avec les acides minéraux ou organiques ou avec les bases, caractérisés en ce qu'ils répondent à la formule générale (I) :

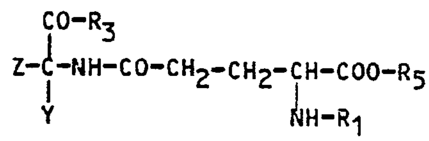

(I)

dans laquelle l'acide glutamique est de configuration D ou L, $R_1$ représente un atome d'hydrogène, un acide aminé ou un peptide constitué de 2, 3 ou 4 acides aminés ou un acide aminé ou un peptide constitué de 2, 3 ou 4 acides aminés dont l'amine est estérifiée par un acide aliphatique saturé ou insaturé $C_6$-$C_{24}$ ou $R_1$ représente le reste d'un acide aliphatique saturé ou insaturé $C_6$-$C_{24}$, $R_5$ représente un atome d'hydrogène ou un radical alkyle renfermant de 1 à 5 atomes de carbone, $R_3$ représente un radical hydroxy, alkoxy $C_1$-$C_5$ ou un acide aminé dont l'amine peut être substituée par un radical alkyle $C_1$-$C_5$, Z représente un groupement de formule :

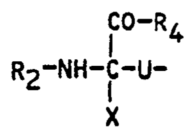

dans lequel $R_2$ représente un atome d'hydrogène, un acide aminé ou un peptide constitué de 2, 3 ou 4 acides aminés, $R_4$ représente un radical hydroxy, alkoxy $C_1$-$C_5$ ou un acide aminé dont l'amine peut être substituée par un radical alkyle $C_1$-$C_5$, U représente une chaîne

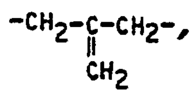, $-CH=CH-CH_2-$(E ou Z), $-CH_2-CH=CH-$(E ou Z) ou

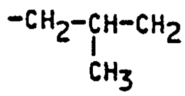

ou U représente ensemble avec X une chaîne $=CH-CH_2-CH_2-$(E ou Z) ou U représente ensemble avec Y une chaîne $-CH_2-CH_2-CH=$ (E ou Z), X représente un atome d'hydrogène ou avec U, une chaîne $=CH-CH_2-CH_2$, (E ou Z) et Y représente un atome d'hydrogène ou avec U une chaîne $-CH_2-CH_2-CH=$, (E ou Z).

Dans la formule générale (I) et dans ce qui suit, l'acide aminé est de préférence un acide alpha-aminé et peut être choisi dans le groupe constitué par Ala, Val, Ival, Leu, Ile, Asp, Asn, Glu, Gln, Ser, Thr, Cys, Met, Lys, Arg, Phe, Tyr, Trp, His et Pro, Nva, Nle, Hyp, Orn, ces acides étant sous la forme D ou L ainsi que pr Sar et Gly, tous les acides précédemment nommés pouvant être N-estérifiés ou N-alcoylés dans le cas d'un peptide comprenant 2, 3 ou 4 acides aminés, ceux-ci sont choisis dans le groupe constitué par les acides aminés ci-dessus.

On admettra par convention que les symboles des acides alpha-amino carboxyliques représentent ces acides sous leur configuration D ou L (par exemple, le terme Ala signifie Alanine sous forme D ou sous forme L).

L'acide aliphatique saturé ou insaturé renferme de 6 à 24 atomes de carbone et de préférence de 12 à 22 atomes de carbone ; on peut citer par exemple les acides stéarique, palmitique, laurique, caprylique, myristique, alpha ou gamma-linolénique, linoléique, arachidonique ou docosopentaénoïque.

Le terme radical alkyle $C_1$-$C_5$ désigne, par exemple, les radicaux pentyle, isobutyle, butyle, isopropyle et

de préférence les radicaux propyle, éthyle ou méthyle.

Le terme radical alkoxy $C_1$-$C_5$ désigne, par exemple, les radicaux pentoxy ou butoxy, mais de préférence propoxy, éthoxy ou méthoxy.

Les produits de formule (I) comportent un ou plusieurs atomes de carbone asymétriques et, comme indiqué ci-dessus, l'invention a pour objet lesdits composés de formule (I) sous toutes leurs formes isomères possibles et sous forme de mélanges.

Les sels des dérivés de l'invention peuvent être formés avec des bases organiques et minérales. Parmi les bases minérales, on peut citer les hydroxydes alcalins et alcalino-terreux, tels que par exemple, les hydroxydes de sodium, de potasium, de lithium et de calcium, l'hydroxyde de magnésium ou d'ammonium. Parmi les bases organiques, on peut citer les amines alcoylées substituées ou non substituées, telles que par exemple, la triméthylamine, la méthylamine, la propylamine, la N,N-diméthyléthanolamine ou la tris-(hydroxyméthyl) méthylamine ; on peut citer également les acides aminés basiques tels que, par exemple, la lysine ou l'arginine; on peut citer encore d'autres bases telles que, par exemple, la glucosamine ou la procaïne.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcanesulfoniques tels que les acides méthane et éthanesulfoniques, arylsulfoniques, tels que les acides benzène ou para-toluènesulfoniques et arylcarboxyliques.

Parmi les produits, objet de l'invention, on peut citer notamment les dérivés répondant à la formule (I) ci-dessus ainsi que leurs sels d'addition, caractérisés en ce que dans ladite formule (I) l'acide glutamique est de configuration D.

Parmi ceux-ci, on peut citer plus particulièrement les dérivés caractérisés en ce que dans ladite formule (I), $R_3$ et $R_4$ représentent un radical hydroxy ainsi que ceux dans laquelle $R_5$ représente un atome d'hydrogène et tout particulièrement :

– l'acide 2-amino 6-(gamma-D-glutamylamino) 4-méthylène heptanedioïque,

– l'acide 2-(L-alanylamino) 6-(gamma-D-glutamylamino) 4-méthylène heptanedioïque,

ainsi que leurs sels d'addition.

L'invention a également pour objet un procédé de préparation des dérivés tels que définis par la formule (I) ci-dessus ainsi que de leurs sels, caractérisé en ce que l'on fait réagir un dérivé de l'acide glutamique de formule (II) :

$$HOOC-CH_2-CH_2-\underset{\substack{|\\ NH-R'_1}}{CH}-COO-Alk$$

dans laquelle Alk représente un radical alkyle $C_1$-$C_3$ et $R'_1$ a la signification de $R_1$ déjà indiquée, à l'exception de l'hydrogène ou représente un groupement protecteur d'une amine, avec un dérivé de formule (III) pouvant se présenter sous forme d'isomères séparés ou sous forme de mélanges d'isomères :

$$R'_2 —— NH —— \overset{\substack{COO-Alk_2\\ |}}{\underset{\substack{|\\ X'}}{C}} —— U —— \overset{\substack{COO-Alk_1\\ |}}{\underset{\substack{|\\ Y'}}{C}} —— NH_2 \qquad (III)$$

dans laquelle U a la signification déjà indiquée, $Alk_1$ et $Alk_2$ représentent un radical alkyle $C_1$-$C_3$, X' et Y' représentent un atome d'hydrogène, un radical $-COO-Alk_3$, $Alk_3$ ayant la signification de $Alk_1$ et $Alk_2$ ou une liaison supplémentaire avec U et $R'_2$ représente un groupement protecteur d'une amine ou un acide aminé ou un peptide constitué de 2, 3 ou 4 acides aminés dont l'amine est protégée par un groupement protecteur, pour obtenir un produit de formule ($I_A$) :

$$R'_2-NH-\overset{\substack{COO-Alk_2\\ |}}{\underset{\substack{|\\ X'}}{C}} —— U —— \overset{\substack{COO-Alk_1\\ |}}{\underset{\substack{|\\ Y'}}{C}}-NH-CO-CH_2-CH_2-\underset{\substack{|\\ NH-R'_1}}{CH}-COO-Alk \qquad (I_A)$$

sous toutes les formes isomères ou sous forme de mélanges d'isomères dans laquelle $R'_1$, $R'_2$, Alk, $Alk_1$, $Alk_2$, X', Y' et U ont la signification déjà indiquée, que l'on isole et, si désiré, salifie, ou l'on soumet si nécessaire et si désiré, à l'une des réactions suivantes, dans un ordre quelconque :

a) si X' ou Y' représente un radical COO-$Alk_3$ : décarboxylation,

b) déprotection des fonctions amino,

c) désalkylation des fonctions hydroxyles,

d) amidification des fonctions amino libres par un acide aminé ou un peptide constitué de 2, 3 ou 4 acides aminés dont la fonction amino est protégée, puis déprotection de cette fonction amino,

e) estérification ou salification par une base des fonctions carboxy,

f) salification par acide des fonctions amino.

Dans des conditions préférentielles de mise en oeuvre, le dérivé de l'acide glutamique est activé par la présence d'agents de condensation tels que le dicyclohexylcarbodiimide, le N,N'-carbonyldiimidazole ou des amides bis-alcoyliques d'acide sulfureux tels que $SO\text{-}(N(CH_3)_2)_2$ ou encore par formation d'anhydride mixte avec le chloroformiate d'isobutyle.

La décarboxylation est de préférence réalisée par l'action d'un acide minéral concentré tel que l'acide chlorhydrique 12 N.

Une décarboxylation directe peut être réalisée directement par la procédure de Krapcho, ou de Keinan et Eren (J. Org. Chem., 51, (1986) 3615-3169.

La déprotection des fonctions amino (formyle ou BOC par exemple) est réalisée de préférence par action d'un acide minéral dilué tel que l'acide chlorhydrique.

La désalkylation des fonctions carboxyles est réalisée de préférence par saponification à l'aide d'une base minérale comme la potasse et notamment la soude. Elle peut, si nécessaire, être opérée en deux fois au cas où elle serait incomplète. Dans ce dernier cas, la seconde désalkylation est de préférence réalisée après la déprotection des fonctions amino et/ou la décarboxylation.

L'amidification des fonctions amino libres est réalisée par exemple à l'aide d'un dérivé fonctionnel tel qu'un halogénure de l'acide aminé ou du peptide ou en présence d'un agent de condensation tel que ceux ci-dessus.

L'estérification des fonctions carboxyles est réalisée par exemple dans les mêmes conditions que ci-dessus (agents de condensation).

Les dérivés de formule (I) présentent selon le cas un caractère basique ou acide. On peut avantageusement préparer les sels d'addition des dérivés de formule (I) en faisant réagir en proportions sensiblement stoéchiométriques, selon le cas, un acide ou une base minérale ou organique, avec ledit dérivé de formule (I).

Dans des conditions préférentielles, l'on effectue sur le dérivé de formule ($I_A$), les opérations suivantes et dans cet ordre :

– désalkylation des fonctions carboxyles,

– déprotection des fonctions amino et décarboxylation,

– amidification ou salification par un acide des fonctions amino et/ou estérification ou salification par une base des fonctions carboxy.

Certains dérivés de formule (III) sont connus. Lorsqu'ils ne sont pas connus, ils peuvent être préparés comme suit :

On fait réagir un dérivé de formule (IV) :

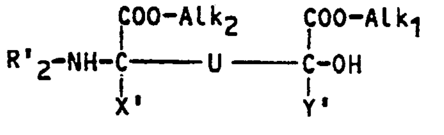

(IV)

dans laquelle $Alk_1$, $Alk_2$, U, X', Y' et $R'_2$ ont la signification déjà indiquée, avec un halogénure d'alcane $C_1$-$C_3$ sulfonyle, de préférence le chlorure de méthanesulfonyle, en présence d'un agent de condensation telle que la pyridine, puis avec un azoture alcalin comme l'azoture de sodium ou le diphénylphosphorylazide, pour obtenir un dérivé de formule (V) :

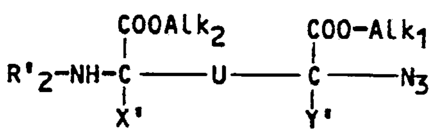

(V)

dans laquelle $Alk_1$, $Alk_2$, U, X', Y' et $R'_2$ ont la signification déjà indiquée, que l'on réduit, par exemple, par action de triphénylphosphine suivie d'hydrolyse aqueuse, ou hydrogénation catalytique, par exemple en présence de palladium sur charbon empoisonné (catalyseur de Lindlar) pour obtenir le dérivé de formule (III) recherché :

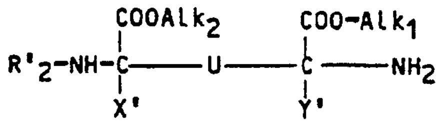 (III)

dans laquelle $R'_2$, X', Y', U, $Alk_1$ et $Alk_2$ ont la signification déjà indiquée, produit de formule (III) que, dans le cas où X' et/ou Y' ne représentent pas un atome d'hydrogène, l'on soumet à l'action d'un réactif de protection du radical amino pour obtenir un produit de formule (III') :

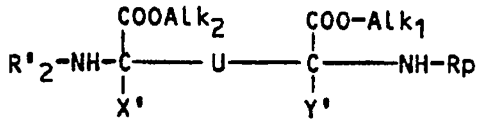 (III')

dans laquelle $R'_2$, $Alk_1$, $Alk_2$, U, X' et Y' ont la signification déjà indiquée et Rp représente un groupement protecteur du radical amino que l'on soumet à l'action d'un réactif de décarboxylation, pour obtenir un produit correspondant comportant 1 ou 2 carbones asymétriques dont on sépare, le cas échéant, les isomères ou diastéréoisomères par les techniques usuelles, par exemple, par cristallisation ou chromatographie, puis soumet à un réactif de déprotection du radical amino pour obtenir le produit de formule (III) recherché sous forme d'isomères séparés ou de mélanges.

Les dérivés de formule (IV) et de formule (II) sont connus. Ils peuvent notamment être préparés comme indiqué dans les exemples.

Les dérivés, objet de la présente invention, possèdent de très intéressantes propriétés pharmacologiques; ils sont doués notamment de remarquables propriétés immunomodulatrices, notamment par activation des monocytes humaines et production de monokines telles que TNF (Tumor Necrosis Factor) et l'IL1 : Interleukine 1.

Ces propriétés sont illustrées plus loin dans la partie expérimentale.

Ces propriétés justifient l'utilisation des dérivés de l'acide glutamique ainsi que de leurs sels, à titre de médicaments.

La présente demande a ainsi également pour objet l'application, à titre de médicaments, des dérivés de l'acide glutamique, tels que définis par la formule (I), sous toutes leurs formes isomères ou sous forme de mélanges d'isomères, ainsi que de leurs sels d'addition pharmaceutiquement acceptables.

Parmi les médicaments, objet de l'invention, on retient de préférence les médicaments caractérisés en ce qu'ils sont constitués par les nouveaux dérivés de l'acide glutamique répondant à la formule (I) dans laquelle l'acide glutamique est de configuration D ainsi que par leurs sels d'addition pharmaceutiquement acceptables.

Parmi ceux-ci, on retient notamment ceux répondant à la formule (I) dans laquelle $R_3$ et $R_4$ représentent un radical hydroxy ainsi que ceux dans laquelle $R_5$ représente un atome d'hydrogène ainsi que leurs sels d'addition pharmaceutiquement acceptables.

Parmi ces derniers, on retient tout particulièrement les dérivés dont les noms suivent :

– l'acide 2-amino 6-(gamma-D-glutamylamino) 4-méthylène heptanedioïque,

– l'acide 2-(L-alanylamino) 6-(gamma-D-glutamylamino) 4-méthylène heptanedioïque,

ainsi que leurs sels pharmaceutiquement acceptables.

Les médicaments selon l'invention trouvent leur emploi, par exemple, dans le traitement des maladies autoimmunes, qu'il s'agisse d'atteintes non spécifiques d'organes (polyarthrite rhumatoïde, lupus érythémateux, anémie hémolytique, leucopénie autoimmune, etc...) ou qu'il s'agisse de maladies spécifiques d'organes (thyroïdite, maladie de Basedow, d'Addison, sclérose en plaques, pemphigus, rectocolite hémorragique, certaines néphropathies, etc...). Ces médicaments peuvent également être utilisés dans le traitement des hémopathies, du cancer, du sida, des affections virales et microbiennes, surtout chroniques et récurrentes (bronchite, grippe, etc...) ; des maladies des cavités buccales, etc.... Ils peuvent constituer des adjuvants de la thérapie virale, de l'antibiothérapie ou de la chimiothérapie anticancéreuse.

Ils trouvent également leur application dans le traitement de nombreux déficits immunitaires secondaires ou acquis observés au cours d'affections très diverses : déficits associés aux troubles métaboliques, déficits

d'origine iatrogène, (corticoïdes, radiations ionisantes...) déficits observés chez les grands brûlés, etc....

La dose usuelle, variables selon le dérivé utilisé, le sujet et l'affection en cause peut être par exemple de 0,05 mg à 50 mg par jour. Par voie orale, chez l'homme, le dérivé de l'exemple 1 peut être administré à la dose quotidienne de 0,5 mg à 50 mg, par exemple, pour le traitement de la polyarthrite rhumatoïde, soit environ de 0,007 mg à 0,7 mg par kilogramme de poids corporel.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) sous toutes leurs formes isomères ou sous forme de mélanges d'isomères et leurs sels d'addition avec les acides ou les bases pharmaceutiquement acceptables peuvent être incorporés dans des compositions pharmaceutiques destinées à la voie digestive ou parentérale.

Ces compositions pharmaceutiques peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les exemples qui suivent, illustrent la présente invention sans toutefois la limiter.

**Exemple 1 : Acide 2-amino 6-(gamma-D-glutamylamino) 4-méthylène heptanedioïque.**

**Stade A :** 1-formylamino 3-méthylène 5-[N-trifluoroacétyl gamma-D-glutamyl (0-méthyl) amino] 1,1,5-pentane tricarboxylate de triéthyle.

On ajoute une solution de 4 g de N-trifluoroacétyl D-glutamate de 1-méthyle dans 20 cm3 de diméthoxyéthane à une solution de 5,1 g de 5-amino 1-(formylamino) 3-méthylène 1,1,5-pentane tricarboxylate de triéthyle dans 280 cm3 de diméthyléthylamine, refroidit à 0°C, ajoute peu à peu 3,2 g de dicyclohexylcarbodiimide, agite pendant 16 heures à 0°C, filtre, amène à sec, reprend le résidu au diméthoxyéthane à froid, filtre, amène à sec, reprend au chlorure de méthylène, lave à l'acide chlorhydrique N, avec une solution saturée de bicarbonate de soude puis à l'eau saturée au chlorure de sodium, sèche, évapore à sec, purifie par chromatographie sur silice (éluant : acétate d'éthyle-cycloheane 5-5) et obtient 5 g du produit attendu.
$[alpha]_D$ = +8° (c = 1% dans le chlorure de méthylène).

**Stade B :** Acide 2-amino 6-(gamma-D-glutamylamino) 4-méthylène heptanedioïque.

Saponification :

On ajoute goutte à goutte en 5 minutes à 0°C, 10,9 cm3 de soude N à une solution de 1,28 g de produit du stade A dans 20 cm3 d'éthanol, laisse revenir à température ambiante, agite pendant 24 heures, neutralise à l'aide de 0,9 cm3 d'acide chlorhydrique 12 N et amène à sec sous pression réduite.

Décarboxylation – Déformylation :

On reprend le résidu dans 10 cm3 d'éthanol, ajoute 1 cm3 d'acide chlorhydrique 12 N et agite 40 minutes à 80°C.

Saponification :

On glace le produit ci-dessus, ajoute goutte à goutte en 5 minutes, 15,2 cm3 de soude 2 N, agite pendant 24 heures à température ambiante, ramène à pH 6 à l'aide d'acide chlorhydrique 12 N et amène à sec.

Purification :

On reprend le produit ci-dessus dans quelques cm3 d'eau, ajuste le pH à 6 à la soude N, déalcalinise sur Résine Dowex 50 W × $8H^+$ (50-100 mesh), on élue d'abord à l'eau puis à l'ammoniaque N, purifie par chromatographie sur silice (éluant : éthanol-ammonique 95-5 puis éthanol-ammoniaque 8-2), amène à sec, reprend

dans 200 cm3 d'eau, filtre, lyophilise pendant 16 heures et obtient 400 mg du produit attendu.
$[alpha]_D$ = −9,5° ± 1° (c = 1% dans l'acide chlorhydrique 4 N).

**A) Préparation du N-trifluoroacétyl D-glutamate de 1-méthyle.**

Stade 1 : N-(trifluoroacétyl) D-glutamate de 5-(1,1-diméthyléthyl) 1-méthyle.

On ajoute 4,1 cm3 de triéthylamine puis en refroidissant et goutte à goutte 4,1 cm3 d'anhydride trifluoroacétique à une solution de 5,5 g de D-glutamate de 5-(1,1-diméthyléthyl) 1-méthyle dans 180 cm3 de chlorure de méthylène. On agite ensuite pendant 2 heures à température ambiante, verse sur 70 cm3 d'eau glacée, extrait au chlorure de méthylène, lave à l'eau salée, sèche, amène à sec et obtient 7,1 g du produit attendu.
$[alpha]_D$ = +5° ± 1° (c = 1,3% dans le dioxanne).

Stade 2 : Préparation du N-trifluoroacétyl D-glutamate de 1-méthyle.

On dissout 6,8 g du produit ci-dessus dans 50 cm3 de chlorure de méthylène, ajoute 50 cm3 d'acide trifluoroacétique, agite pendant 1 heure, amène à sec, reprend à l'acétate d'éthyle, verse sur de l'eau, extrait à l'acétate d'éthyle, lave à l'eau salée, amène à sec et obtient 5,5 g du produit attendu.
$[alpha]_D$ = +9° ± 2° (c = 0,6% dans le dioxanne).

**B) Préparation du 5-amino 1-(formylamino) 3-méthylène 1,1,5-pentane tricarboxylate de triéthyle de départ de l'exemple 1.**

Stade 1 : 2-(formylamino) (2-méthyl 2-propényl) propanedioate de diéthyle.

On ajoute 40 g de carbonate de potassium, 0,380 g de catalyseur éther couronne 18 crown 6 et 39,9 g de chlorométhyl propène à une solution de 30 g de formamido-malonate d'éthyle dans 300 cm3 de cyanométhyle, agite au reflux pendant 3 heures, filtre, amène à sec, refroidit à 0°-5°C, reprend dans 10 cm3 d'éther isopropylique, filtre, lave à l'éther isopropylique, sèche sous pression réduite et obtient 25,2 g du produit attendu.
F ≃ 72°C.

Stade 2 : 1-(formylamino) 5-hydroxy 3-méthylène 1,1,5-pentane tricarboxylate de triéthyle (mélange d'isomères).

On ajoute goutte à goutte en 10 minutes une solution de 26,5 g de glyoxylate d'éthyle dans 180 cm3 de chlorure de méthylène, à une solution de 84 g de chlorure ferrique dans 180 cm3 de chlorure de méthylène, agite 1 heure, refroidit à −20°C, ajoute goutte à goutte en 20 minutes une solution de 34 g de produit tel qu'obtenu au stade A dans 180 cm3 de chlorure de méthylène et agite 1 heure à −20°C. On verse sur 300 cm3 d'eau glacée, extrait au chlorure de méthylène, lave avec HCl 2 N puis à l'eau salée, sèche, amène à sec sous pression réduite et obtient 56,5 g du produit attendu (mélange d'isomères). F ≃ 68°C après purification par chromatographie sur silice (éluant : acétate d'éthyle-cyclohexane 6-4).

Stade 3 : 5-azido 1-(formylamino) 3-méthylène 1,1,5-pentane tricarboxylate de triéthyle.

On dissout 46 g du produit (mélange d'isomères) obtenu au stade 2 dans 500 cm3 de pyridine, refroidit à 0°C, ajoute goutte à goutte à 0°C, 12 cm3 de chlorure de méthanesulfonyle, agite 3 heures à température ambiante, verse sur 400 cm3 d'acide chlorhydrique 4 N glacé et 200 cm3 de chlorure de méthylène, extrait au chlorure de méthylène, lave avec de l'acide chlorhydrique 4 N avec une solution saturée de bicarbonate de soude et avec de l'eau salée, sèche et évapore à sec sous pression réduite. On dissout les 56 g de résidu obtenus dans 250 cm3 de diméthylformamide, ajoute 9,98 g d'azoture de sodium, agite 16 heures à température ambiante, élimine le solvant, reprend dans du chlorure de méthylène, lave avec une solution saturée en bicarbonate de sodium puis à l'eau salée, sèche, amène à sec et obtient 59 g de produit attendu (mélange d'isomères que l'on purifie par chromatographie sur silice (éluant : cyclohexane-acétate d'éthyl 8-2) pour obtenir 26 g du produit attendu.

Stade 4 : 5-amino 1-(formylamino) 3-méthylène 1,1,5-pentane tricarboxylate de triéthyle.

On ajoute 10,9 g de triphénylphosphine à une solution de 13 g du produit ci-dessus dans 250 cm3 de tétrahydrofuranne à environ −5°C, agite 5 heures à température ambiante, ajoute 8,5 cm3 d'eau, agite 24 heures à température ambiante, évapore le tétrahydrofuranne, reprend le résidu au chlorure de méthylène, verse sur de l'acide chlorhydrique 2 N glacé, extrait à l'acide chlorhydrique 2 N, neutralise au bicarbonate de sodium, extrait au chlorure de méthylène, lave à l'eau salée, sèche, amène à sec et obtient 10 g du produit attendu. F 50°C après recristallisation dans l'éther isopropylique.

**Exemple 2 : Acide 2-(L-alanylamino) 6-(gamma-D-glutamylamino) 4-méthylène heptanedioïque**

Stade A : 1-amino 3-méthylène 5-[N-trifluoroacétyl gamma-D-glutamyl (0-méthyl) amino] 1,1,5-pentane tricarboxylate de triéthyle.

On ajoute 1,5 cm3 d'acide chlorhydrique 12 N à une solution de 1,22 g de produit du stade A de l'exemple 1 dans 15 cm3 d'éthanol, agite 40 minutes à 80°C, élimine le solvant sous pression réfuite, reprend le résidu dans 10 cm3 d'eau, neutralise avec du bicarbonate de sodium, extrait au chlorure de méthylène, lave à l'eau salée, sèche, amène à sec, purifie par chromatographie sur silice (éluant : acétate d'éthyle-cyclohexane 8-2) et obtient 0,850 g du produit attendu.

Stade B : 1-[N-[(1,1-diméthyléthoxy) carbonyl] L-alanylamino] 3-méthylène 5-[N-trifluoroacétyl gamma-D-glutamyl (0-méthyl) amino] 1,1,5-pentane tricarboxylate de triéthyle.

On dissout 0,310 g de B.O.C.-L-alanine dans 50 cm3 de tétrahydrofuranne, ajoute 0,43 cm3 de triéthylamine, refroidit à 5°C environ, ajoute 0,23 cm3 de chloroformiate d'isobutyle, agite 30 minutes à +5°C environ, ajoute goutte à goutte en 5 minutes une solution de 0,800 g de produit du stade A dans 10 cm3 de tétrahydrofuranne, laisse revenir à température ambiante et agite 16 heures. On élimine le tétrahydrofuranne, reprend dans un mélange eau-chlorure de méthylène, extrait au chlorure de méthylène, lave à l'eau salée, sèche, amène à sec, purifie par chromatographie sur silice (éluant : acétate d'éthyle-cyclohexane 5-5) et obtient le produit attendu. $[alpha]_D = 12° \pm 1°$ (c = 1% dans le chlorure de méthylène).

Stade C : Acide 2-(L-alanylamino) 6-(gamma-D-glutamylamino) 4-méthylène heptanedioïque.

On opère comme indiqué ci-dessus au stade B de l'exemple 1, à partir de 700 mg de produit du stade B ci-dessus.
Saponification, déprotection de l'amine protégée par le B.O.C., saponification, purification et obtient 170 mg du produit attendu.
$[alpha]_D -13° \pm 1°$ (c = 1% HCl 4 N).

**Exemple 3 : Acide 2-amino 4-méthylène 6-[N-(1-oxo octadécyl) L-alanyl gamma-D-glutamylamino] heptanedioïque.**

On opère comme indiqué à l'exemple 1 ci-dessus, mais en utilisant le N-[N-(1-oxo octadécyl) L-alanyl] D-glutamate de 1-méthyle à la place du N-trifluoroacétyle correspondant, obtenu dans la préparation A de l'exemple 1, pour obtenir le produit attendu.
$[alpha]_D = -25°$ (c = 0,6% dans l'eau).

**Préparation du N-[N-(1-oxo octadécyl) L-alanyl] D-glutamate de 1-méthyle.**

Stade 1 : N-(1-oxo octadécyl) L-alaninate de méthyle.

A une solution de 10 g de chlorhydrate de L-analine méthyl ester dans 250 cm3 de chlorure de méthylène, on ajoute 24,9 cm3 de triéthylamine, puis goutte à goutte en 30 minutes, une solution de 21,7 g de chlorure de stéaryle dans 50 cm3 de chlorure de méthylène, agite pendant 2 heures 30 minutes, filtre, lave avec de l'acide chlorhydrique 2 N, puis à l'eau salée, sèche, amène à sec sous pression réduite, empâte le résidu à l'éther isopropylique, essore et recristallise dans le méthanol. On obtient 22 g du produit attendu. F ≃ 84°C. $[alpha]_D = -15°$ (c = 1% pyridine).

Stade 2 : N-(1-oxo octadécyl) L-alanine.

On refroidit à 0°C une suspension de 21 g de produit ci-dessus dans 500 cm3 de méthanol, ajoute goutte

à goutte 62 cm3 de potasse N, agite pendant 16 heures à température ambiante, ajoute 500 cm3 de méthanol, agite pendant 4 heures, refroidit à 0°C, amène à pH 3 à l'aide d'acide chlorhydrique 2 N, évapore, reprend au chlorure de méthylène, lave à l'eau salée, sèche, amène à sec sous pression réduite, empâte le résidu à l'éther isopropylique, essore, recristallise dans le méthanol et obtient 11,8 g du produit attendu. F 102°C.
$[alpha]_D$ = −18° (c = 0,9% pyridine).

<u>Stade 3</u> : N-[N-(1-oxo octadécyl) L-alanyl] D-glutamate de 5-(1,1-diméthyléthyle) et 1-méthyle.

On refroidit à 0°C une suspension de 1,77 g de N-(1-oxo octadécyl) L-alanine et 1,08 g de D-glutamate de 5-(1,1-diméthyléthyle- et 1-méthyle dans 170 cm3 de diméthoxyéthane, ajoute goutte à goutte en 10 minutes une solution de 1,23 g de N,N-dicyclohexylcarbodiimide dans 5 cm3 de diméthoxy éthane, agite 3 heures à température ambiante, refroidit, filtre, amène à sec, reprend dans 250 cm3 d'éther à chaud, filtre, amène à sec sous pression réduite, empâte dans l'éther isopropylique, essore et obtient 2,4 g du produit attendu. F ≃ 100°C après recristallisation dans le méthanol.
$[alpha]_D$ = −32° ± 1° (c = 1% $CH_2Cl_2$).

<u>Stade 4</u> : N-[N-(1-oxo octadécyl) L-alanyl] D-glutamate de 1-méthyle.

On ajoute goutte à goutte 31 cm3 d'acide trifuoroacétique à une solution de 11,4 g du produit ci-dessus dans 150 cm3 de chlorure de méthylène, laisse pendant 16 heures sous agitation, amène à sec sous pression réduite, reprend au chlorure de méthylène, lave à l'eau salée, sèche, filtre, amène à sec sous pression rédite, cristallise, recristallise dans l'éthanol et obtient 6,8 g du produit attendu. F ≃ 110°C.
$[alpha]_D$ = −16° ± 1° (c = 0,9% pyridine).

**<u>Exemple 4</u> : <u>Acide 2-amino 6-(gamma-D-glutamylamino) 3-heptènedioïque.</u>**

On opère comme indiqué à l'exemple 1 ci-dessus, mais en utilisant du 5-amino 1-formylamino 1,1,5-pent-2-ène tricarboxylate de triéthyle, pour obtenir le produit attendu.

**Préparation du 5-amino 1-formylamino 2-pent-2-èn-1,1,5-tricarboxylate de triéthyle.**

On opère comme indiqué ci-dessus pour la préparation du 5-amino 1-(formylamino) 3-méthylène 1,1,5-pentane tricarboxylate de triéthyle, mais en remplaçant le chlorométhyl propène par du bromure d'allyle, pour obtenir le produit attendu sous forme d'une huile. Rf = 0,4 dans l'acétate d'éthyle-éthanol 4-1.

**<u>Exemple 5</u> : <u>Acide 2-glycylamino 6-[[N-(1-oxododécyl) L-alanyl gamma-D-glutamyl] amino] 2-heptènedioïque.</u>**

On opère comme indiqué aux exemples 1 et 3 en faisant réagir le N-[N-(1-oxododécyl) L-alanyl] D-glutamate de 1-méthyle préparé comme le N-[N-(1-oxododécyl) L-alanyl] D-glutamate de 1-méthyle à l'exemple 3 en remplaçant le stéaryle par le lauryle (dodécyle) avec le 6-amino 2-[(N-formylglycyl) amino 3-heptènedioate de 7-éthyl 1-méthyle). Dans ce cas, la déformylation par l'acide chlorhydrique n'est pas nécessaire. La neutralisation et la désalification peuvent se faire simultanément à l'aide d'une résine $H^+$ (Amberlyst 15). On obtient le produit attendu devenant gommeux à 150°C.
$[alpha]_D$ = −14° (c = 0,8% dans l'eau).

**<u>Exemple 6</u> : <u>Mélange des acides (2D, 6L) et (2L, 6D) 2-amino 6-(gamma-D-glutamylamino) 4-méthylène heptanedioïque.</u>**

<u>Stade A</u> : Mélange d'acides (2D, 6L) et (2L, 6D) 2-formylamino 4-méthylène 6-[N-(trifluoroacétyl) gamma-D-glutamylamino] heptanedioïque.

On ajoute à 0°C, 1,3 g de dicyclohexylcarbodiimide dans une solution comprenant 1,5 g du mélange (2D, 6L) et (2L, 6D) 2-formamido 4-méthylène 6-amino 1,7-heptanedioate de diéthyle et 1,48 g d'acide D-glutamique dans 150 cm3 de diméthoxyéthane et agite 16 heures en maintenant la température à 0°C. On filtre le précipité et concentre à sec le filtrat sous pression réduite. On obtient 10,5 g de produit brut que l'on purifie par chromatographie sur silice (éluant : cyclohexane-acétate d'éthyle 2-8).
$[alpha]_D$ = −14° (c = 0,8% dans l'eau).

Stade B : Mélange des acides (2D, 6L) et (2L, 6D) 2-amino 6-(gamma-D-glutamylamino) 4-méthylène heptanedioïque.

On ajoute 5 cm3 d'acide chlorhydrique 12 N à 1,9 g de produit obtenu au stade A en solution dans 100 cm3 d'éthanol et agite 30 minutes. On élimine le solvant sous pression réduite, reprend le résidu dans 50 cm3 d'éthanol, ajuste le pH à 7 à l'aide d'une solution de soude 0,1 N, refroidit à 0°C et ajoute goutte à goutte 16 cm3 de soude N. On laisse revenir à température ambiante, agite 16 heures, neutralise le milieu réactionnel avec de l'acide chlorhydrique N puis élimine les solvants sous pression réduite. On chromatographie le résidu sur silice (éluant : éthanol-ammoniaque 95-5, 90-10 puis 80-20), reprend la résine obtenue dans l'eau, filtre et lyophilise. On obtient 0,92 g de produit attendu.
$[alpha]_D$ = −13° ± 1° (c = 1% HCl 3 N).

**Exemple 7 : Mélange des acides (2D, 6D) et (2L, 6L) 2-amino 6-(gamma-D-glutamylamino) 4-méthylène heptanedioïque.**

Stade A : Mélange d'acides (2D, 6D) et (2L, 6L) 2-formylamino 4-méthylène 6-[N-(trifluoroacétyl) gamma-D-glutamylamino.

On opère comme au stade A de l'exemple 6 à partir du mélange (2D, 6D) et (2L, 6L) 2-formamido 4-méthylène 6-amino 1,7-heptanedioate de diéthyle.

Stade B : Mélange des acides (2D, 6D) et (2L, 6L) 2-amino 6-(gamma-D-glutamylamino) 4-méthylène heptanedioïque.

On opère comme au stade B de l'exemple 6 à partir du produit obtenu au stade A ci-dessus.
$[alpha]_D$ = −12,5° ± 1° (c = HCl 3 N).

Le mélange (2D, 6D) et (2L, 6L) 2-formamido 4-méthylène 6-amino 1,7-heptanedioate de diéthyle utilisé au départ de l'exemple 7 a été préparé comme indiqué à la préparation de l'exemple (Stade C), en utilisant au départ le mélange de (2D, 6D) et de (2L, 6L) formamido 4-méthylène 6-terbutoxy carbonylamino 1,7-heptanedioate de diéthyle (isomère I) obtenu au stade B de cette préparation.

**Préparation du 6-amino 2-[(N-formylglycyl) amino] 3-hepténedioate de 7-éthyl 1-méthyle.**

On opère comme indiqué à la préparation B de l'exemple 1 en remplaçant au stade 2, le (formylamino) (2-méthyl 2-propényl) propanedioate de diéthyle par le 2-[(N-formylglycyl) amino] 2-(2-propényl) éthaneoate de méthyle, pour obtenir le produit attendu. Rf = 0,2 dans le mélange chlorure de méthylène-méthanol (9-1).

Remarque : La réduction de l'azoture en amine (stade 4 de la préparation B de l'exemple 1) est réalisée dans ce cas par hydrogénation en présence de palladium sur carbonate de calcium et de quinoléine, au lieu de triphénylphosphine.

**Préparation du mélange de (2D, 6L) et (2L, 6D) 2-formamido 4-méthylène 6-amino 1,7-heptanedioate de diéthyle.**

Stade A : 1-formamido 3-méthylène 5-(terbutoxycarbonylamino) 1,1,5-pentane tricarboxylate de triéthyle.

On ajoute à température ambiante 13,6 g de pyrocarbonate de diterbutyle en solution dans 25 cm3 de chlorure de méthylène à 19,5 g de 1-formamido 3-méthylène 5-amino 1,1,5-pentane tricarboxylate de triéthyle en solution dans 75 cm3 de chlorure de méthylène et maintient sous agitation à température ambiante pendant 36 heures. On dilue le milieu réactionnel avec 150 cm3 de chlorure de méthylène, lave la phase organique à l'eau, la sèche et élimine les solvants sous pression réduite, laisse cristalliser à +4°C et recueille 25,8 g de produit attendu. F = 68°C.

Stade B : Mélange de (2D, 6D) et de (2L, 6L) 2-formamido 4-méthylène 6-(terbutoxycarbonylamino) 1,7-heptanedioate de diéthyle et mélange d'isomères (2D, 6L) et (2L, 6D).

On ajoute 2,21 g de diéthyle de césium et 9,42 g de para-aminophénol à 17,25 g de produit obtenu au stade A en solution dans 250 cm3 de diméthyl formamide et chauffe pendant 3 heures à 85°C. On laisse revenir à température ambiante, filtre et élimine les solvants sous pression réduite. On reprend le résidu dans 300 cm3

de chlorure de méthylène, lave la phase organique avec de l'acide chlorhydrique 0,5 N puis avec une solution aqueuse de bicarbonate de soude, sèche et élimine le solvant sous pression réduite et chromatographie sur colonne de silice (éluant : cyclohexane-acétate d'éthyle 6-4) et obtient 4,70 g d'isomère I (isomère DD, LL), 3,84 g d'isomère II (isomère DL, LD) et 2,80 g de mélange des 2 isomères.

Stade C : Mélange de (2D, 6L) et de (2L, 6D) 2-formamido 4-méthylène 6-amino 1,7-heptanedioate de diéthyle.

On ajoute 25 cm3 d'acide chlorhydrique 2 N dans l'éther à 3 g de produit II obtenu au stade B (isomère DL, LD) en solution dans 50 cm3 de chlorure de méthylène. On agite à température ambiante pendant 30 minutes, concentre sous atmosphère inerte, reprend le chlorhydrate de l'amine dans le chlorure de méthylène additionné de triéthylamine puis amène à sec sous pression réduite. Après chromatographie sur silice (éluant : acétate d'éthylecyclohexane 7-3), on récupère 1,86 g de produit attendu.

Exemple 8 :

On a préparé des comprimés répondant à la formule :

- Acide 2-amino 6-(gamma-D-glutamylamino) 4-méthylène heptadioïque ...................................................... 50 mg
- Excipient q.s. pour un comprimé terminé à .................... 100 mg.
(Détail de l'excipient : lactose, amidon, talc, stéarate de magnésium).

Exemple 9 :

On a préparé des comprimés répondant à la formule :

- Acide 2-(L-alanylamino) 6-(gamma-D-glutamylamino) 4-méthylène heptadioïque ...................................................... 50 mg
- Excipient q.s. pour un comprimé terminé à .................... 100 mg.
(Détail de l'excipient : lactose, amidon, talc, stéarate de magnésium).

ETUDE PHARMACOLOGIQUE

Stimulation des cellules monocytaires par un immunostimulant

Les cellules mononuclées du sang circulant de donneurs normaux sont séparées suivant la technique classique décrite par Boyum utilisant un gradient de Ficoll. Après lavage, les mononucléaires sont mis à incuber à 37°C pendant 1 heure à raison de $5.10^6$ monocytes (cellules $NSE^+$) par ml de milieu de culture, 5 ml par flacon de culture. Le milieu de culture utilisé dans cette expérience se compose de RPMI 1640 additionné d'antibiotiques et de tampon Hépès. Au bout d'une heure, les cellules non adhérentes sont enlevées par lavage des flacons à l'aide de milieu précédement porté à 37°C, les cellules adhérentes se composant essentiellement de monocytes (> 90%) sont remises en culture en présence de différentes quantités de produits à tester dans un milieu P.B.S. (Dulbeno) sans $Ca^{2+}$ ni$Mg^{2+}$. La culture est poursuivie pendant 24 ou 48 heures et les surnageants des cellules sont alors prélevés, centrifugés, aliquotés et conservés soit à –80°C soit à –20°C. Les surnageants de culture sont remplacés, dans les flacons par la même quantité d'eau distillée apyrogène afin de lyser les cellules. Le lysat est récupéré, aliquoté et conservé également à –20°C. Les expériences suivantes ont été réalisées en présence ou en l'absence d'Interferon gamma ($10^3$ U/ml) à une dose où l'IFN gamma seul est inactif.

**Tests de la présence dans les surnageants de monokines (Interleukine-1 et Tumor Necrosis Factor) par leur activité biologique.**

Test Interleukine-1 (IL-1) :

Ce test a été décrit la première fois par I. Gery et Waksman en 1972 (Gery I. and B.H. Waksman, 1972. Potentiation of the T lymphocyte response to miteogens. II. The cellular source of potentialing mediator (s) J. Exp. Med., 136-143).

Il est basé sur l'action co-mitogène de l'IL-1 en présence d'un antigène (mimé par la phytohémaglutinine dans le test) sur des thymocytes de souris. 1,5 × $10^6$ thymocytes de souris C3H/HeJ (provenant du C.S.E.A.L. d'Orléans) sont mis en culture pendant 3 jours en présence de différentes dilutions de surnageants et de lysats cellulaires susceptibles de contenir de l'activité IL-1 et de PHA-P Wellcome (1 µg/ml) dans des plaques de culture 96 puits fond plat, dans un volume final de 200 µl de milieu composé de RPMI 1640 contenant outre des antibiotiques (pénicilline 1/ml – steptomycine 1000 U/ml de tampon Hépès 1 mM de la glutamine, 2 mM 5% de sérum de veau et 5 × $10^{-5}$ M de 2-mercaptoéthanol. Au bout de 68 heures de culture, 1 µCi de thymidine tritiée est ajoutée à chaque puits ($^3$H-méthyl-thymidine, CEA Saclay, TMM79A activité spécifique 1 uCi/mM), la radioactivité incorporée par les cellules est évaluée après avoir filtré les cultures sur appareil collecteur semi-automatique de type Skaton et compté les filtres dans un compteur à scintillation (LKB). Les résultats sont exprimés par la différence entre les impulsions par minute incorporées par les cultures en présence de surnageants et les impulsions par minute incorporées par les cultures témoins.

Test Tumor Necrosis Factor (TNF) :

L'activité TNF est mise en évidence par la toxicité de ce facteur sur des cellules cibles L-929 (sous-clone alpha). La technique a été sensibilisée en ajoutant dans le test de l'actinomycine D.

Les cellules L sont distribuées à raison de 2 × $10^4$ cellules par puits d'une microplaque à fond plat dans 100 µl de milieu RPMI 1640 enrichi avec 5% de sérum de veau, de la glutamine, du tampon Hépès et des antibiotiques. Au bout de 24 heures, différents dilutions des surnageants à tester sont ajoutés dans un volume de 100 ul ainsi qu'une dose d'actinomycine D de 1 µg/ml. Au bout de 24 heures de culture, la quantité de cellules viables non lysées est évaluée en colorant les plaques au violet crystal et en mesurant la densité optique des différents puits sur lecteur multiscan.

RESULTATS :

Les produits des exemples 1 et 2 stimulent les monocytes et leur production d'IL-1 et de TNF. De plus, il y a sunergie entre les produits des exemples 1 et 2 et l'Interféron gamma.

## Revendications

### Revendications pour les Etats contractants : AT, BE, CH, DE, FR, GB, IT, LI, LU, NL, SE

1. Sous toutes leurs formes isomères ou sous formes de mélanges d'isomères des dérivés de l'acide glutamique ainsi que leurs sels d'addition avec les acides minéraux ou organiques ou avec les bases, caractérisés en ce qu'ils répondent à la formule générale (I) :

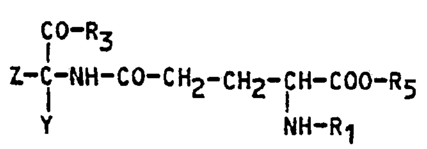

(I)

dans laquelle l'acide glutamique est de configuration D ou L, $R_1$ représente un atome d'hydrogène, un acide aminé ou un peptide constitué de 2, 3 ou 4 acides aminés ou un acide aminé ou un peptide constitué de 2, 3 ou 4 acides aminés dont l'amine est estérifiée par un acide aliphatique saturé ou insaturé $C_6$-$C_{24}$ ou $R_1$ représente le reste d'un acide aliphatique saturé ou insaturé $C_6$-$C_{24}$, $R_5$ représente un atome d'hydrogène ou un radical alkyle renfermant de 1 à 5 atomes de carbone, $R_3$ représente un radical hydroxy, alkoxy $C_1$-$C_5$ ou un acide aminé dont l'amine peut être substituée par un radical alkyle $C_1$-$C_5$, Z représente un groupement de formule :

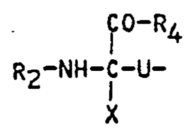

dans lequel $R_2$ représente un atome d'hydrogène, un acide aminé ou un peptide constitué de 2, 3 ou 4 acides aminés, $R_4$ représente un radical hydroxy, alkoxy $C_1$-$C_5$ ou un acide aminé dont l'amine peut être substituée par un radical alkyle $C_1$-$C_5$, U représente une chaîne

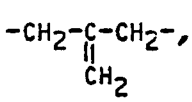 $-CH_2-CH=CH-CH_2-$ (E ou Z), $-CH_2-CH=CH-$ (E ou Z) ou

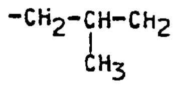

ou U représente ensemble avec X une chaîne $=CH-CH_2-CH_2-$(E ou Z) ou U représente ensemble avec Y une chaîne $-CH_2-CH_2-CH=$ (E ou Z), X représente un atome d'hydrogène ou avec U, une chaîne $=CH-CH_2-CH_2$, (E ou Z) et Y représente un atome d'hydrogène ou avec U une chaîne $-CH_2-CH_2-CH=$, (E ou Z).

2. Les dérivés de l'acide glutamique tels que définis par la formule (I) de la revendication 1, ainsi que leurs sels d'addition, caractérisés en ce que l'acide glutamique est de configuration D.

3. Les dérivés de l'acide glutamique selon la revendication 2, ainsi que leurs sels d'addition, caractérisés en ce que dans ladite formule (I), $R_3$ et $R_4$ représentent un radical hydroxy.

4. Les dérivés de l'acide glutamique selon la revendication 2 ou 3 ainsi que leurs sels d'addition, caractérisés en ce que $R_5$ représente un atome d'hydrogène.

5. Les dérivés dont les noms suivent :

– l'acide 2-amino 6-(gamma-D-glutamylamino) 4-méthylène heptanedioïque,

– l'acide 2-(L-alanylamino) 6-(gamma-D-glutamylamino) 4-méthylène heptanedioïque,

ainsi que leurs sels d'addition.

6. Procédé de préparation des dérivés tels que définis par la formule (I) de la revendication 1 ainsi que de leurs sels, caractérisé en ce que l'on fait réagir un dérivé de l'acide glutamique de formule (II) :

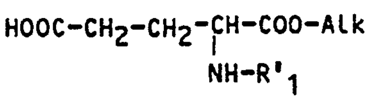 (II)

dans laquelle Alk représente un radical alkyle $C_1$-$C_3$ et $R'_1$ a la signification de $R_1$ déjà indiquée, à l'exception de l'hydrogène ou représente un groupement protecteur d'une amine, avec un dérivé de formule (III) pouvant se présenter sous forme d'isomères séparés ou sous forme de mélanges d'isomères :

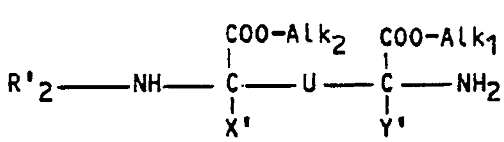 (III)

dans laquelle U a la signification déjà indiquée, $Alk_1$ et $Alk_2$ représentent un radical alkyle $C_1$-$C_3$, X' et Y' représentent un atome d'hydrogène, un radical $-COO-Alk_3$, $Alk_3$ ayant la signification de $Alk_1$ et $Alk_2$ ou une liaison supplémentaire avec U et $R'_2$ représente un groupement protecteur d'une amine ou un acide aminé ou un peptide constitué de 2, 3 ou 4 acides aminés dont l'amine est protégée par un groupement protecteur, pour obtenir un produit de formule ($I_A$) :

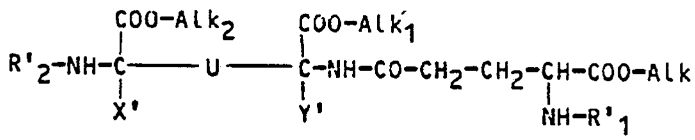

($I_A$)

sous toutes les formes isomères ou sous forme de mélanges d'isomères dans laquelle $R'_1$, $R'_2$, Alk, $Alk_1$, $Alk_2$, X', Y' et U ont la signification déjà indiquée, que l'on isole et, si désiré, salifie, ou l'on soumet si nécessaire et si désiré, à l'une des réactions suivantes, dans un ordre quelconque :

a) si X' ou Y' représente un radical $COO\text{-}Alk_3$ : décarboxylation,

b) déprotection des fonctions amino,

c) désalkylation des fonctions hydroxyles,

d) amidification des fonctions amino libres par un acide aminé ou un peptide constitué de 2, 3 ou 4 acides aminés dont la fonction amino est protégée, puis déprotection de cette fonction amino,

e) estérification ou salification par une base des fonctions carboxy,

f) salification par acide des fonctions amino.

7. Procédé selon la revendication 6, caractérisé en ce que l'on effectue sur le dérivé de formule ($I_A$), les opérations suivantes et dans cet ordre :

– désalkylation des fonctions carboxyles,

– déprotection des fonctions amino et décarboxylation,

– amidification ou salification par un acide des fonctions amino et/ou estérification ou salification par une base des fonctions carboxy.

8. Médicaments, caractérisés en ce qu'ils sont constitués par les nouveaux dérivés de l'acide glutamique, tels que définis par la formule (I) de la revendication 1 sous toutes leurs formes isomères ou sous forme de mélanges d'isomères, ainsi que par leurs sels d'addition avec les acides pharmaceutiquement acceptables.

9. Médicaments, caractérisés en ce qu'ils sont constitués par les nouveaux dérivés de l'acide glutamique, tels que définis dans l'une des revendications 2, 3 ou 4, ainsi que par leurs sels d'addition avec les acides pharmaceutiquement acceptables.

10. Médicaments, caractérisés en ce qu'ils sont constitués par les nouveaux dérivés de l'acide glutamique, tels que définis à la revendication 5, ainsi que par leurs sels d'addition pharmaceutiquement acceptables.

11. Compositions pharmaceutiques, caractérisées en ce qu'elles renferment, à titre de principe actif, l'un au moins des médicaments, tels que définis à l'une des revendications 8, 9 ou 10.

**Revendications pour l'Etat contractant suivant : ES**

1. Procédé de préparation sous toutes leurs formes isomères ou sous formes de mélanges d'isomères des dérivés de l'acide glutamique ainsi que leurs sels d'addition avec les acides minéraux ou organiques ou avec les bases, caractérisés en ce qu'ils répondent à la formule générale (I) :

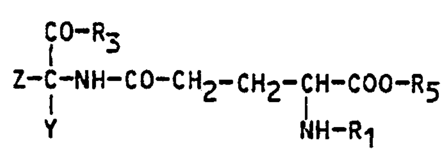

(I)

dans laquelle l'acide glutamique est de configuration D ou L, $R_1$ représente un atome d'hydrogène, un acide aminé ou un peptide constitué de 2, 3 ou 4 acides aminés ou un acide aminé ou un peptide constitué de 2, 3 ou 4 acides aminés dont l'amine est estérifiée par un acide aliphatique saturé ou insaturé $C_6$-$C_{24}$ ou $R_1$ représente le reste d'un acide aliphatique saturé ou insaturé $C_6$-$C_{24}$, $R_5$ représente un atome d'hydrogène ou un radical alkyle renfermant de 1 à 5 atomes de carbone, $R_3$ représente un radical hydroxy, alkoxy $C_1$-$C_5$ ou un acide aminé dont l'amine peut être substituée par un radical alkyle $C_1$-$C_5$, Z représente un groupement de formule :

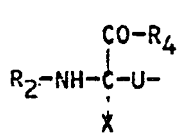

dans lequel $R_2$ représente un atome d'hydrogène, un acide aminé ou un peptide constitué de 2, 3 ou 4 acides aminés, $R_4$ représente un radical hydroxy, alkoxy $C_1$-$C_5$ ou un acide aminé dont l'amine peut être substituée par un radical alkyle $C_1$-$C_5$, U représente une chaîne

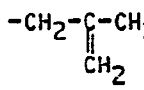, $-CH=CH-CH_2-$(E ou Z), $-CH_2-CH=CH-$(E ou Z) ou

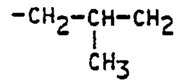

ou U représente ensemble avec X une chaîne $=CH-CH_2-CH_2-$(E ou Z) ou U représente ensemble avec Y une chaîne $-CH_2-CH_2-CH=$ (E ou Z), X représente un atome d'hydrogène ou avec U, une chaîne $=CH-CH_2-CH_2$, (E ou Z) et Y représente un atome d'hydrogène ou avec U une chaîne $-CH_2-CH_2-CH=$, (E ou Z), caractérisé en ce que l'on fait réagir un dérivé de l'acide glutamique de formule (II) :

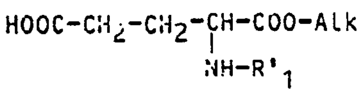

(II)

dans laquelle Alk représente un radical alkyle $C_1$-$C_3$ et $R'_1$ a la signification de $R_1$ déjà indiquée, à l'exception de l'hydrogène ou représente un groupement protecteur d'une amine, avec un dérivé de formule (III) pouvant se présenter sous forme d'isomères séparés ou sous forme de mélanges d'isomères :

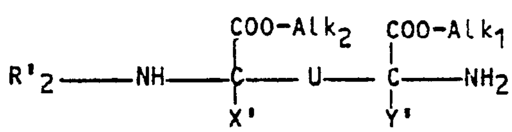

(III)

dans laquelle U a la signification déjà indiquée, $Alk_1$ et $Alk_2$ représentent un radical alkyle $C_1$-$C_3$, X' et Y' représentent un atome d'hydrogène, un radical $-COO-Alk_3$, $Alk_3$ ayant la signification de $Alk_1$ et $Alk_2$ ou une liaison supplémentaire avec U et $R'_2$ représente un groupement protecteur d'une amine ou un acide aminé ou un peptide constitué de 2, 3 ou 4 acides aminés dont l'amine est protégée par un groupement protecteur, pour obtenir un produit de formule ($I_A$) :

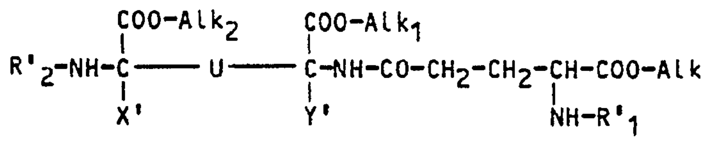

($I_A$)

sous toutes les formes isomères ou sous forme de mélanges d'isomères dans laquelle $R'_1$, $R'_2$, Alk, $Alk_1$, $Alk_2$, X', Y' et U ont la signification déjà indiquée, que l'on isole et, si désiré, salifie, ou l'on soumet si nécessaire et si désiré, à l'une des réactions suivantes, dans un ordre quelconque :

a) si X' ou Y' représente un radical $COO-Alk_3$ : décarboxylation,

b) déprotection des fonctions amino,

c) désalkylation des fonctions hydroxyles,

d) amidification des fonctions amino libres par un acide aminé ou un peptide constitué de 2, 3 ou 4 acides aminés dont la fonction amino est protégée, puis déprotection de cette fonction amino,

e) estérification ou salification par une base des fonctions carboxy,

f) salification par acide des fonctions amino.

2. Procédé selon la revendication 1, caractérisé en ce que l'on effectue sur le dérivé de formule ($I_A$), les opérations suivantes et dans cet ordre :

– désalkylation des fonctions carboxyles,

– déprotection des fonctions amino et décarboxylation,
– amidification ou salification par un acide des fonctions amino et/ou estérification ou salification par une base des fonctions carboxy.

3. Procédé selon la revendication 1 ou 2, caractérisé en ce que l'on utilise au départ un dérivé de l'acide glutamique de formule (II) de configuration D.

4. Procédé selon la revendication 1, 2 ou 3, caractérisé en ce que l'on prépare un produit de formule (I) dans laquelle $R_3$ et $R_4$ représentent un radical hydroxy.

5. Procédé selon l'une quelconque des revendications 1 à 4, caractérisé en ce que l'on prépare un produit de formule (I) dans laquelle $R_5$ représente un atome d'hydrogène.

6. Procédé selon l'une quelconque des revendications 1 à 5, caractérisé en ce que l'on prépare un produit de formule (I) dont les noms suivent :
– l'acide 2-amino 6-(gamma-D-glutamylamino) 4-méthylène heptanedioïque,
– l'acide 2-(L-alanylamino) 6-(gamma-D-glutamylamino) 4-méthylène heptanedioïque,
ainsi que leurs sels d'addition.

**Revendications pour l'Etat contractant suivant : GR**

1. Procédé de préparation sous toutes leurs formes isomères ou sous formes de mélanges d'isomères des dérivés de l'acide glutamique ainsi que leurs sels d'addition avec les acides minéraux ou organiques ou avec les bases, caractérisés en ce qu'ils répondent à la formule générale (I) :

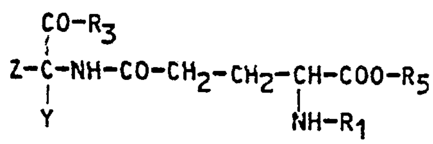

(I)

dans laquelle l'acide glutamique est de configuration D ou L, $R_1$ représente un atome d'hydrogène, un acide aminé ou un peptide constitué de 2, 3 ou 4 acides aminés ou un acide aminé ou un peptide constitué de 2, 3 ou 4 acides aminés dont l'amine est estérifiée par un acide aliphatique saturé ou insaturé $C_6$-$C_{24}$ ou $R_1$ représente le reste d'un acide aliphatique saturé ou insaturé $C_6$-$C_{24}$, $R_5$ représente un atome d'hydrogène ou un radical alkyle renfermant de 1 à 5 atomes de carbone, $R_3$ représente un radical hydroxy, alkoxy $C_1$-$C_5$ ou un acide aminé dont l'amine peut être substituée par un radical alkyle $C_1$-$C_5$, Z représente un groupement de formule :

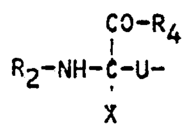

dans lequel $R_2$ représente un atome d'hydrogène, un acide aminé ou un peptide constitué de 2, 3 ou 4 acides aminés, $R_4$ représente un radical hydroxy, alkoxy $C_1$-$C_5$ ou un acide aminé dont l'amine peut être substituée par un radical alkyle $C_1$-$C_5$, U représente une chaîne

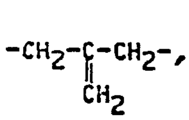, $-CH=CH-CH_2-$(E ou Z), $-CH_2-CH=CH-$(E ou Z) ou

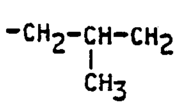

ou U représente ensemble avec X une chaîne $=CH-CH_2-CH_2-$(E ou Z) ou U représente ensemble avec Y une chaîne $-CH_2-CH_2-CH=$ (E ou Z), X représente un atome d'hydrogène ou avec U, une chaîne $=CH-CH_2-CH_2$, (E ou Z) et Y représente un atome d'hydrogène ou avec U une chaîne $-CH_2-CH_2-CH=$, (E ou Z), caractérisé en ce que l'on fait réagir un dérivé de l'acide glutamique de formule (II) :

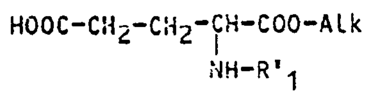

(II)

dans laquelle Alk représente un radical alkyle $C_1$-$C_3$ et $R'_1$ a la signification de $R_1$ déjà indiquée, à l'exception de l'hydrogène ou représente un groupement protecteur d'une amine, avec un dérivé de formule (III) pouvant se présenter sous forme d'isomères séparés ou sous forme de mélanges d'isomères :

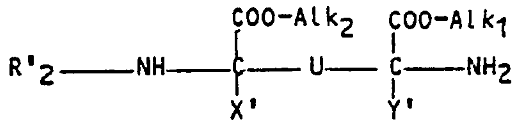

(III)

dans laquelle U a la signification déjà indiquée, $Alk_1$ et $Alk_2$ représentent un radical alkyle $C_1$-$C_3$, X' et Y' représentent un atome d'hydrogène, un radical –COO-$Alk_3$, $Alk_3$ ayant la signification de $Alk_1$ et $Alk_2$ ou une liaison supplémentaire avec U et $R'_2$ représente un groupement protecteur d'une amine ou un acide aminé ou un peptide constitué de 2, 3 ou 4 acides aminés dont l'amine est protégée par un groupement protecteur, pour obtenir un produit de formule ($I_A$) :

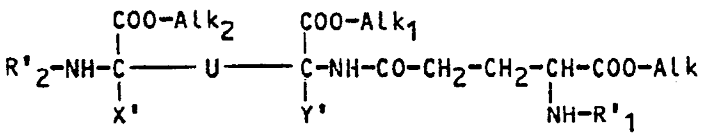

($I_A$)

sous toutes les formes isomères ou sous forme de mélanges d'isomères dans laquelle $R'_1$, $R'_2$, Alk, $Alk_1$, $Alk_2$, X', Y' et U ont la signification déjà indiquée, que l'on isole et, si désiré, salifie, ou l'on soumet si nécessaire et si désiré, à l'une des réactions suivantes, dans un ordre quelconque :

a) si X' ou Y' représente un radical COO-$Alk_3$ : décarboxylation,
b) déprotection des fonctions amino,
c) désalkylation des fonctions hydroxyles,
d) amidification des fonctions amino libres par un acide aminé ou un peptide constitué de 2, 3 ou 4 acides aminés dont la fonction amino est protégée, puis déprotection de cette fonction amino,
e) estérification ou salification par une base des fonctions carboxy,
f) salification par acide des fonctions amino.

2. Procédé selon la revendication 1, caractérisé en ce que l'on effectue sur le dérivé de formule ($I_A$), les opérations suivantes et dans cet ordre :

– désalkylation des fonctions carboxyles,
– déprotection des fonctions amino et décarboxylation,
– amidification ou salification par un acide des fonctions amino et/ou estérification ou salification par une base des fonctions carboxy.

3. Procédé selon la revendication 1 ou 2, caractérisé en ce que l'on utilise au départ un dérivé de l'acide glutamique de formule (II) de configuration D.

4. Procédé selon la revendication 1, 2 ou 3, caractérisé en ce que l'on prépare un produit de formule (I) dans laquelle $R_3$ et $R_4$ représentent un radical hydroxy.

5. Procédé selon l'une quelconque des revendications 1 à 4, caractérisé en ce que l'on prépare un produit de formule (I) dans laquelle $R_5$ représente un atome d'hydrogène.

6. Procédé selon l'une quelconque des revendications 1 à 5, caractérisé en ce que l'on prépare un produit de formule (I) dont les noms suivent :

– l'acide 2-amino 6-(gamma-D-glutamylamino) 4-méthylène heptanedioïque,
– l'acide 2-(L-alanylamino) 6-(gamma-D-glutamylamino) 4-méthylène heptanedioïque,

ainsi que leurs sels d'addition.

7. Procédé de préparation de compositions pharmaceutiques, caractérisé en ce que l'on met à titre de principe actif l'un au moins des produits de formule (I), telle que définie à la revendication 1, sous toutes ses formes isomères ou sous forme de mélanges d'isomères ou l'un au moins de ses sels d'addition avec les acides minéraux ou organiques ou avec les bases, sous une forme destinée à cet usage.

8. Procédé de préparation de compositions pharmaceutiques, caractérisé en ce que l'on met à titre de principe actif l'un au moins des produits de formule (I), telle que définie à la revendication 6, sous toutes ses formes isomères ou sous forme de mélanges d'isomères ou l'un au moins de ses sels d'addition avec les acides minéraux ou organiques ou avec les bases, sous une forme destinée à cet usage.

## Claims

### Claims for the Contracting States : AT, BE, CH, DE, FR, GB, IT, LI, LU, NL, SE.

1. The derivatives of glutamic acid in all their isomer forms or in the form of isomer mixtures as well as their addition salts with mineral or organic acids or with bases, characterized in that they correspond to general formula (I) :

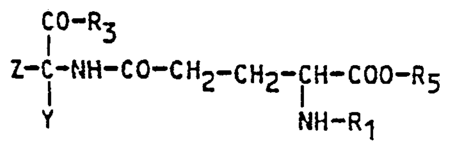

(I)

in which the glutamic acid is of D or L configuration, $R_1$ represents a hydrogen atom, an amino acid or a peptide composed of 2, 3 or 4 amino acids or an amino acid or a peptide composed of 2, 3 or 4 amino acids, the amine of which is esterified by a saturated or unsaturated $C_6$-$C_{24}$ aliphatic acid or $R_1$ represents the remainder of a saturated or unsaturated $C_6$-$C_{24}$ aliphatic acid, $R_5$ represents a hydrogen atom or an alkyl radical containing 1 to 5 carbon atoms, $R_3$ represents a hydroxy radical, a $C_1$-$C_5$ alkoxy radical or an amino acid, the amine of which can be substituted by a $C_1$-$C_5$ alkyl radical, Z represents a group of formula :

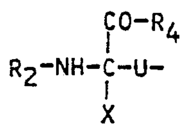

in which $R_2$ represents a hydrogen atom, an amino acid or a peptide composed of 2, 3 or 4 amino acids, $R_4$ represents a hydroxy radical, a $C_1$-$C_5$ alkoxy radical or an amino acid, the amine of which can be substituted by a $C_1$-$C_5$ alkyl radical, U represents a

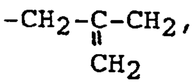, $-CH=CH-CH_2-$(E or Z), $CH_2-CH=CH-$(E or Z) or

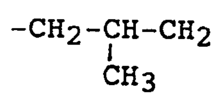

chain or U represents together with X a $=CH-CH_2-CH_2$ (E or Z) chain or U represents together with Y a $-CH_2-CH_2-CH=$ (E or Z) chain, X represents a hydrogen atom or with U a $=CH-CH_2-CH_2$ (E or Z) chain and Y represents a hydrogen atom or with U a $-CH_2-CH_2-CH=$ chain (E or Z).

2. The derivatives of glutamic acid as defined by formula (I) of claim 1, as well as their addition salts, characterized in that the glutamic acid is of D configuration.

3. The derivatives of glutamic acid according to claim 2, as well as their addition salts, characterized in that in said formula (I), $R_3$ and $R_4$ represent a hydroxy radical.

4. The derivatives of glutamic acid according to claim 2 or 3 as well as their addition salts, characterized in that $R_5$ represents a hydrogen atom.

5. The derivatives of which the names follow :

– 2-amino-6-(gamma-D-glutamylamino)-4-methylene heptanedioic acid,

– 2-(L-alanylamino)-6-(gamma-D-glutamylamino)-4-methylene heptanedioic acid,

as well as their addition salts.

6. Preparation process for the derivatives as defined by formula (I) of claim 1 as well as their addition salts, characterized in that a derivative of glutamic acid of formula (II) :

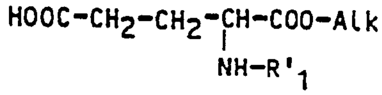

(II)

in which Alk represents a $C_1$-$C_3$ alkyl radical and $R'_1$ has the meaning already indicated for $R_1$, with the exception of the hydrogen, or represents an amine protector group, is reacted with a derivative of formula (III) being able to be in separated isomer form or in the form of a mixture of isomers :

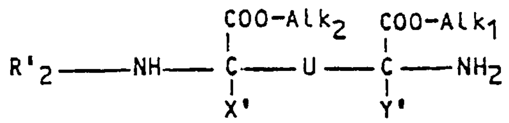

(III)

in which U has the meaning already indicated, $Alk_1$ and $Alk_2$ represent a $C_1$-$C_3$ alkyl radical, X' and Y' represent a hydrogen atom, a $-COO-Alk_3$ radical, $Alk_3$ having the meaning of $Alk_1$ and $Alk_2$ or a supplementary bond with U and $R'_2$ represents an amine protector group or an amino acid or a peptide composed of 2, 3 or 4 amino acids the amine of which is protected by a protector group, in order to obtain a product of formula ($I_A$) :

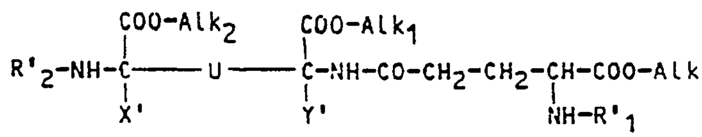

($I_A$)

in all their isomer forms or in the form of isomer mixtures in which $R'_1$, $R'_2$, Alk, $Alk_1$, $Alk_2$, X', Y' and U have the meaning already indicated, which is isolated and, if desired, salified, or if necessary and if desired, is subjected to one of the following reactions, in any order :

a) if X' and Y' represent a $COO-Alk_3$ radical : decarboxylation,
b) deprotection of the amino functions,
c) dealkylation of the hydroxyl functions,
d) amidification of the free amino functions by an amino acid or a peptide composed of 2, 3 or 4 amino acids of which the amino function is protected, then deprotection of this amino function,
e) esterification or salification by a base of the carboxy functions,
f) salification by an acid of the amino functions.

7. Process according to claim 6, characterized in that the following operations are carried out on the derivative of formula ($I_A$) and in this order :

- dealkylation of the carboxyl functions,
- deprotection of the amino functions and decarboxylation,
- amidification or salification by an acid of the amino functions and/or esterification or salification by a base of the carboxy functions.

8. Medicaments, characterized in that they are constituted by the new derivatives of glutamic acid, as defined by formula (I) of claim 1 in all their isomer forms or in the form of isomer mixtures, as well as by their addition salts with pharmaceutically acceptable acids.

9. Medicaments, characterized in that they are constituted by the new derivatives of glutamic acid, as defined in one of claims 2, 3 or 4, as well as by their addition salts with pharmaceutically acceptable acids.

10. Medicaments, characterized in that they are constituted by the new derivatives of glutamic acid, as defined in claims 5, as well as by their pharmaceutically acceptable addition salts.

11. Pharmaceutical compositions, characterized in that they contain, as active ingredient, at least one of the medicaments, as defined in one of claims 8, 9 or 10.

**Claims for the contracting State : ES**

1. Preparation process for the derivatives of glutamic acid in all their isomer forms or in the form of isomer

mixtures as well as their addition salts with mineral or organic acids or with bases, characterized in that they correspond to general formula (I) :

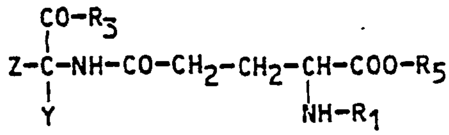

(I)

in which the glutamic acid is of D or L configuration, $R_1$ represents a hydrogen atom, an amino acid or a peptide composed of 2, 3 or 4 amino acids or an amino acid or a peptide composed of 2, 3 or 4 amino acids the amine of which is esterified by a saturated or unsaturated $C_6$-$C_{24}$ aliphatic acid or $R_1$ represents the remainder of a saturated or unsaturated $C_6$-$C_{24}$ aliphatic acid, $R_5$ represents a hydrogen atom or an alkyl radical containing 1 to 5 carbon atoms, $R_3$ represents a hydroxy radical, a $C_1$-$C_5$ alkoxy radical or an amino acid the amine of which can be substituted by a $C_1$-$C_5$ alkyl radical, Z represents a group of formula :

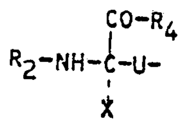

in which $R_2$ represents a hydrogen atom, an amino acid or a peptide composed of 2, 3 or 4 amino acids, $R_4$ represents a hydroxy radical, a $C_1$-$C_5$ alkoxy radical or an amino acid the amine of which can be substituted by a $C_1$-$C_5$ alkyl radical, U represents a

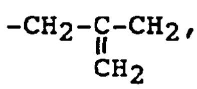, $-CH=CH-CH_2-$(E or Z), $CH_2-CH=CH-$(E or Z) or

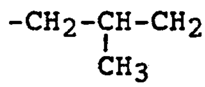

chain or U represents together with X a $=CH-CH_2-CH_2$ (E or Z) chain or U represents together with Y a $-CH_2-CH_2-CH=$ (E or Z) chain, X represents a hydrogen atom or with U a $=CH-CH_2-CH_2$ (E or Z) chain and Y represents a hydrogen atom or with U a $-CH_2-CH_2-CH=$ (E or Z) chain, characterized in that a derivative of glutamic acid of formula (II) :

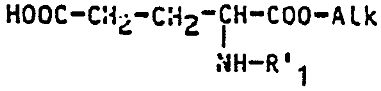

(II)

in which Alk represents a $C_1$-$C_3$ alkyl radical and $R'_1$ has the meaning already indicated for $R_1$, with the exception of the hydrogen or represents an amine protector group, is reacted with a derivative of formula (III) being able to be in separated isomer form or in the form of a mixture of isomers :

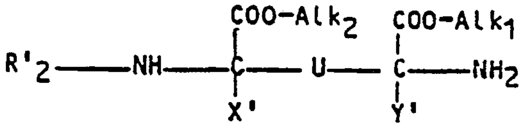

(III)

in which U has the meaning already indicated, $Alk_1$ and $Alk_2$ represent a $C_1$-$C_3$ alkyl radical, X' and Y' represent a hydrogen atom, a $-COO-Alk_3$ radical, $Alk_3$ having the meaning of $Alk_1$ and $Alk_2$ or a supplementary bond with U and $R'_2$ represents an amine protector group or an amino acid or a peptide composed of 2, 3 or 4 amino acids the amine of which is protected by a protector group, in order to obtain a product of formula ($I_A$) :

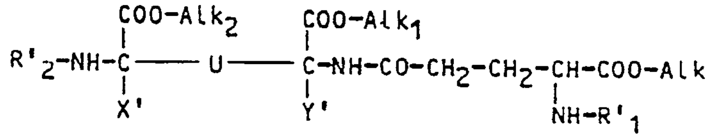

($I_A$)

in all the isomer forms or in the form of isomer mixtures in which $R'_1$, $R'_2$, Alk, $Alk_1$, $Alk_2$, X', Y' and U have the meaning already indicated, which is isolated and, if desired, salified, or if necessary and if desired, is subjected to one of the following reactions, in any order :

a) if X' or Y' represents a $COO\text{-}Alk_3$ radical : decarboxylation,

b) deprotection of the amino functions,

c) dealkylation of the hydroxyl functions,

d) amidification of the free amino functions by an amino acid or a peptide composed of 2, 3 or 4 amino acids of which the amino function is protected, then deprotection of this amino function,

e) esterification or salification by a base of the carboxy functions,

f) salification by an acid of the amino functions.

2. Process according to claim 1, characterized in that the following operations are carried out on the derivative of formula ($I_A$) and in this order :

– dealkylation of the carboxyl functions,

– deprotection of the amino functions and decarboxylation,

– amidification or salification by an acid of the amino functions and/or esterification or salification by a base of the carboxy functions.

3. Process according to claim 1 or 2, characterized in that a derivative of glutamic acid of formula (II) of D configuration is used at the start.

4. Process according to claim 1, 2 or 3, characterized in that a product of formula (I), in which $R_3$ and $R_4$ represent a hydroxy radical, is prepared.

5. Process according to any one of claims 1 to 4, characterized in that a product of formula (I) in which $R_5$ represents a hydrogen atom is prepared.

6. Process according to any one of claims 1 to 5, characterized in that a product of formula (I) is prepared, the names of which follow :

– 2-amino-6-(gamma-D-glutamylamino)-4-methylene heptanedioic acid,

– 2-(L-alanylamino)-6-(gamma-D-glutamylamino)-4-methylene heptanedioic acid,

as well as their addition salts.

**Claims for the contractin State : GR**

1. Preparation process for the derivatives of glutamic acid in all their isomer forms or in the form of isomer mixtures as well as their addition salts with mineral or organic acids or with bases, characterized in that they correspond to general formula (I) :

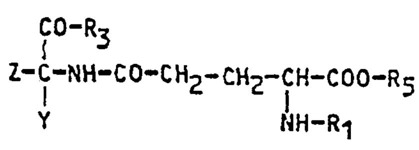

(I)

in which the glutamic acid is of D or L configuration, $R_1$ represents a hydrogen atom, an amino acid or a peptide composed of 2, 3 or 4 amino acids or an amino acid or a peptide composed of 2, 3 or 4 amino acids the amine of which is esterified by a saturated or unsaturated $C_6$-$C_{24}$ aliphatic acid or $R_1$ represents the remainder of a saturated or unsaturated $C_6$-$C_{24}$ aliphatic acid, $R_5$ represents a hydrogen atom or an alkyl radical containing 1 to 5 carbon atoms, $R_3$ represents a hydroxy radical, a $C_1$-$C_5$ alkoxy radical or an amino acid the amine of which can be substituted by a $C_1$-$C_5$ alkyl radical, Z represents a group of formula :

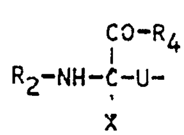

in which $R_2$ represents a hydrogen atom, an amino acid or a peptide composed of 2, 3 or 4 amino acids, $R_4$ represents a hydroxy radical, a $C_1$-$C_5$ alkoxy radical or an amino acid the amine of which can be substituted by a $C_1$-$C_5$ alkyl radical, U represents a

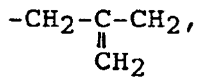 , $-CH=CH-CH_2-$(E or Z), $CH_2-CH=CH-$(E or Z) or

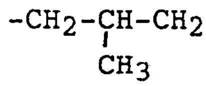

chain or U represents together with X a $=CH-CH_2-CH_2$ (E or Z) chain or U represents together with Y a $-CH_2-CH_2-CH=$ (E or Z) chain, X represents a hydrogen atom or with U a $=CH-CH_2-CH_2$ (E or Z) chain and Y represents a hydrogen atom or with U a $-CH_2-CH_2-CH=$ (E or Z) chain, characterized in that a derivative of glutamic acid of formula (II) :

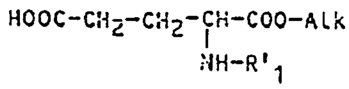

(II)

in which Alk represents a $C_1$-$C_3$ alkyl radical and $R'_1$ has the meaning already indicated for $R_1$, with the exception of the hydrogen or represents an amine protector group, is reacted with a derivative of formula (III) being able to be in separated isomer form or in the form of a mixture of isomers :

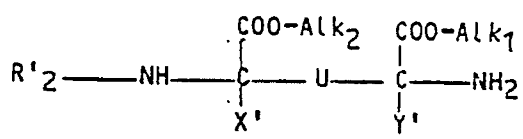

(III)

in which U has the meaning already indicated, $Alk_1$ and $Alk_2$ represent a $C_1$-$C_3$ alkyl radical, X' and Y' represent a hydrogen atom, a $-COO-Alk_3$ radical, $Alk_3$ having the meaning of $Alk_1$ and $Alk_2$ or a supplementary bond with U and $R'_2$ represents an amine protector group or an amino acid or a peptide composed of 2, 3 or 4 amino acids the amine of which is protected by a protector group, in order to obtain a product of formula ($I_A$) :

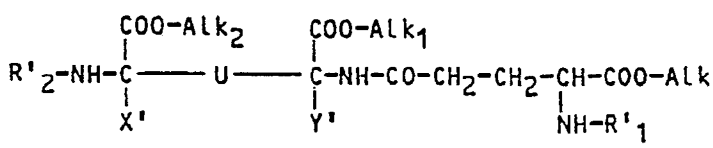

($I_A$)

in all their isomer forms or in the form of isomer mixtures in which $R'_1$, $R'_2$, Alk, $Alk_1$, $Alk_2$, X', Y' and U have the meaning already indicated, which is isolated and, if desired, salified, or if necessary and if desired, is subjected to one of the following reactions, in any order :

a) if X' or Y' represents a $COO-Alk_3$ radical : decarboxylation,

b) deprotection of the amino functions,

c) dealkylation of the hydroxyl functions,

d) amidification of the free amino functions by an amino acid or a peptide composed of 2, 3 or 4 amino acids of which the amino function is protected, then deprotection of this amino function,

e) esterification or salification by a base of the carboxy functions,

f) salification by an acid of the amino functions.

2. Process according to claim 1, characterized in that the following operations are carried out on the derivative of formula ($I_A$) and in this order :

– dealkylation of the carboxyl functions,

– deprotection of the amino functions and decarboxylation,

– amidification or salification by an acid of the amino functions and/or esterification or salification by a base of the carboxy functions.

3. Process according to claim 1 or 2, characterized in that a derivative of glutamic acid of formula (II) of D configuration is used at the start.

4. Process according to claim 1, 2 or 3, characterized in that a product of formula (I), in which $R_3$ and $R_4$ represent a hydroxy radical, is prepared.

5. Process according to any one of claims 1 to 4, characterized in that a product of formula (I) in which $R_5$ represents a hydrogen atom is prepared.

6. Process according to any one of claims 1 to 5, characterized in that a product of formula (I) is prepared, the names of which follow :

– 2-amino-6-(gamma-D-glutamylamino)-4-methylene heptanedioic acid,

– 2-(L-alanylamino)-6-(gamma-D-glutamylamino)-4-methylene heptanedioic acid,

as well as their addition salts.

7. Preparation process for pharmaceutical compositions, characterized in that at least one of the products of formula (I), as defined in claim 1, is used as active ingredient, in all its isomer forms or in the form of isomer mixtures or at least one of its addition salts with mineral or organic acids or with bases, in a form intended for this use.

8. Preparation process for pharmaceutical compositions, characterized in that at least one of the products of formula (I), as defined in claim 6, is used as active ingredient, in all its isomer forms or in the form of isomer mixtures or at least one of its addition salts with mineral or organic acids or with bases, in a form intended for this use.

## Ansprüche

### Patentansprüche für die Vertragsstaaten : AT, BE, CH, DE, FR, GB, IT, LI, LU, NL, SE

1. Derivate der Glutaminsäure sowie ihrer Additionssalze mit Mineral- oder organischen Säuren oder mit Basen unter allen isomeren Formen oder unter den Formen der Isomerengemische, dadurch gekennzeichnet, daß sie der allgemeinen Formel (I)

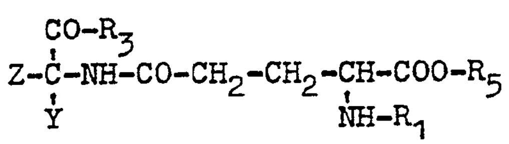

(I)

entsprechen, worin die Glutaminsäure die D- oder L-Konfiguration hat, $R_1$ ein Wasserstoffatom, eine Aminosäure oder ein Peptid, bestehend aus 2, 3 oder 4 Aminosäuren, oder eine Aminosäure oder ein Peptid, bestehend aus 2, 3 oder 4 Aminosäuren, deren Amin durch eine gesättigte oder ungesättigte, aliphatische $C_6$-$C_{24}$-Säure verestert ist, bedeutet oder $R_1$ bedeutet den Rest einer gesättigten oder ungesättigten, aliphatischen $C_6$-$C_{24}$-Säure, $R_5$ bedeutet ein Wasserstoffatom oder einen Alkylrest mit 1 bis 5 Kohlenstoffatomen, $R_3$ bedeutet einen Hydroxy-, $C_1$-$C_5$-Alkoxy-Rest oder eine Aminosäure, deren Amin durch einen $C_1$-$C_5$ -Akylrest substituiert sein kann, Z bedeutet eine Gruppe der Formel

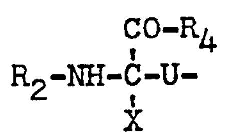

worin $R_2$ ein Wasserstoffatom, eine Aminosäure oder ein Peptid, bestehend aus 2, 3 oder 4 Aminosäuren, bedeutet, $R_4$ bedeutet einen Hydroxy-, $C_1$-$C_5$-Alkoxyrest oder eine Aminosäure, deren Amin durch einen $C_1$-$C_5$-Alkylrest substituiert sein kann, U bedeutet eine Kette

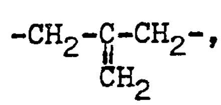

$-CH=CH-CH_2-$(E oder Z), $-CH_2-CH=CH-$(E oder Z) oder

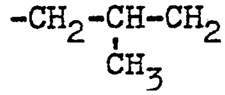

oder U bedeutet zusammen mit X eine Kette $=CH-CH_2-CH_2-$(E oder Z) oder U bedeutet zusammen mit Y eine Kette $-CH_2-CH_2-CH=$ (E oder Z), X bedeutet ein Wasserstoffatom oder zusammen mit U eine Kette $=CH-CH_2-CH_2-$(E oder Z) und Y bedeutet ein Wasserstoffatom oder mit U eine Kette $-CH_2-CH_2-CH=$ (E oder Z).

2. Die Glutaminsäurederivate, wie sie durch Formel (I) von Anspruch 1 definiert sind, sowie ihre Additionssalze, dadurch gekennzeichnet, daß die Glutaminsäure in der D-Konfiguration ist.

3. Derivate der Glutaminsäure gemäß Anspruch 2 sowie ihre Additionssalze, dadurch gekennzeichnet, daß in der Formel (I) $R_3$ und $R_4$ einen Hydroxyrest bedeuten.

4. Derivate der Glutaminsäure gemäß Anspruch 2 oder 3 sowie ihre Additionssalze, dadurch gekennzeichnet, daß $R_5$ ein Wasserstoffatom bedeutet.

5. Die folgenden, namentlichen Derivate :

2-Amino-6-(γ-D-glutamylamino)-4-methylenheptandisäure,

2-(L-Alanylamino)-6-(γ-D-glutamylamino)-4-methylenheptandisäure

sowie ihre Additionssalze.

6. Verfahren zur Herstellung der Derivate, wie sie in Formel (I) in Anspruch 1 definiert sind, sowie ihrer Salze, dadurch gekennzeichnet, daß man ein Derivat der Glutaminsäure der Formel (II)

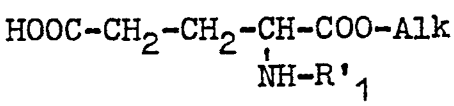 (II)

worin Alk einen $C_1$-$C_3$-Alkylrest bedeutet und $R'_1$ die für $R_1$ angegebene Bedeutung hat mit Ausnahme von Wasserstoff oder eine Schutzgruppe eines Amins bedeutet, mit einem Derivat der Formel (III), das in Form von getrennten Isomeren oder in Form eines Isomerengemisches vorliegen kann :

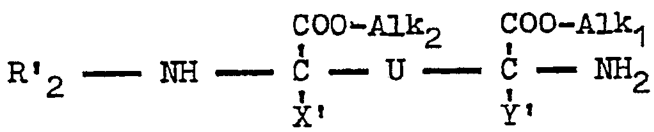 (III)

zur Reaktion bringt, worin U die angegebene Bedeutung hat, $Alk_1$ und $Alk_2$ einen $C_1$-$C_3$-Alkylrest darstellen, X′ und Y′ ein Wasserstoffatom, einen Rest $-COO-Alk_3$, wobei $Alk_3$ die Bedeutung von $Alk_1$ und $Alk_2$ hat, oder eine zusätzliche Bindung mit U bedeuten und $R'_2$ eine Schutzgruppe eines Amins oder eine Aminosäure oder ein Peptid aus 2, 3 oder 4 Aminosäuren bedeutet, deren Amin durch eine Schutzgruppe geschützt ist, um ein Produkt der Formel ($I_A$)

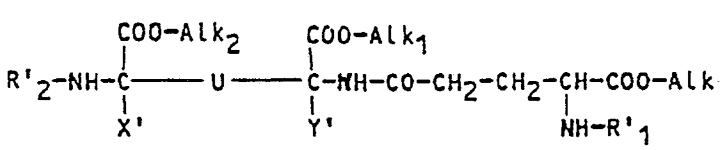 ($I_A$)

in allen seinen isomeren Formen oder in Form von Isomerengemischen zu erhalten, wobei $R'_1$, $R'_2$, Alk, $Alk_1$, $Alk_2$, X′, Y′ und U die angegebene Bedeutung haben, daß man isoliert und gewünschtenfalls in ein Salz überführt oder es, falls notwendig und gewünscht, einer der folgenden Reaktionen in irgendeiner Reihenfolge unterwirft :

a) wenn X′ oder Y′ einen Rest $COO-Alk_3$ bedeuten : Decarboxylierung,

b) Schutzgruppenentfernung der Aminofunktionen,

c) Entalkylierung der Hydroxylfunktionen,

d) Amidierung der freien Aminofunktionen durch eine Aminosäure oder ein Peptid aus 2, 3 oder 4 Aminosäuren, deren Aminofunktion geschützt ist, dann Schutzgruppenentfernung dieser Aminofunktion,

e) Veresterung oder Salzbildung der Carboxylfunktionen durch eine Base,

f) Salzbildung der Aminofunktionen durch eine Säure.

7. Verfahren gemäß Anspruch 6, dadurch gekennzeichnet, daß man an dem Derivat der Formel ($I_A$) die folgenden Arbeitsgänge und in dieser Reihenfolge vornimmt :

– Entalkylierung der Carboxylfunktionen,

– Schutzgruppenentfernung der Aminofunktionen und Decarboxylierung,

– Amidierung oder Salzbildung der Aminofunktionen durch eine Säure und/oder Veresterung oder Salzbildung der Carboxylfunktionen durch eine Base.

8. Arzneimittel, dadurch gekennzeichnet, daß sie aus den neuen Derivaten der Glutaminsäure, wie sie durch die Formel (I) von Anspruch 1 definiert sind, unter allen Isomeren Formen und unter der Form der Isomerengemische bestehen sowie ihren Additionssalzen mit pharmazeutisch annehmbaren Säuren.

9. Arzneimittel, dadurch gekennzeichnet, daß sie aus den neuen Derivaten der Glutaminsäure, wie sie in einem der Ansprüche 2, 3 oder 4 definiert sind, sowie aus ihren Additionssalzen mit pharmazeutisch annehmbaren Säuren bestehen.

10. Arzneimittel, dadurch gekennzeichet, daß die aus den neuen Derivaten der Glutaminsäure, wie sie in Anspruch 5 definiert sind, sowie ihren pharmazeutisch annehmbaren Additionssalzen bestehen.

11. Pharmazeutische Zuammensetzungen, dadurch gekennzeichnet, daß sie als aktives Prinzip wenigstens eines der Arzneimittel, wie sie in den Ansprüchen 8, 9 oder 10 definiert sind, enthalten.

**Patentansprüche für den Vertragsstaat : ES**

1. Verfahren zur Herstellung von Derivaten der Glutaminsäure sowie ihren Additionssalzen mit Mineral- oder organischen Säuren oder mit Basen unter allen isomeren Formen oder unter den Formen der Isomerengemische, dadurch gekennzeichnet, daß sie der allgemeinen Formel (I)

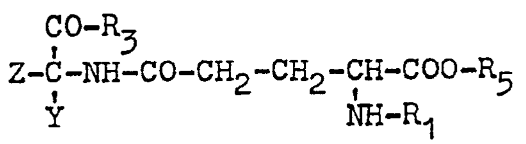

(I)

entsprechen, worin die Glutaminsäure die D- oder L-Konfiguration hat, $R_1$ ein Wasserstoffatom, eine Aminosäure oder ein Peptid, bestehend aus 2, 3 oder 4 Aminosäuren, oder eine Aminosäure oder ein Peptid, bestehend aus 2, 3 oder 4 Aminosäuren, deren Amin durch eine gesättigte oder ungesättigte, aliphatische $C_6$-$C_{24}$-Säure verestert ist, bedeutet oder $R_1$ bedeutet den Rest einer gesättigten oder ungesättigten, aliphatischen $C_6$-$C_{24}$-Säure, $R_5$ bedeutet ein Wasserstoffatom oder einen Alkylrest mit 1 bis 5 Kohlenstoffatomen, $R_3$ bedeutet einen Hydroxy-, $C_1$-$C_5$-Alkoxy-Rest oder eine Aminosäure, deren Amin durch einen $C_1$-$C_5$-Alkylrest substituiert sein kann, Z bedeutet eine Gruppe der Formel

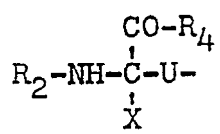

worin $R_2$ ein Wasserstoffatom, eine Aminosäure oder ein Peptid, bestehend aus 2, 3 oder 4 Aminosäuren, bedeutet, $R_4$ bedeutet einen Hydroxy-, $C_1$-$C_5$-Alkoxyrest oder eine Aminosäure, deren Amin durch einen $C_1$-$C_5$-Alkylrest substituiert sein kann, U bedeutet eine Kette

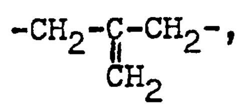

$-CH=CH-CH_2-$(E oder Z), $-CH_2-CH=CH-$(E oder Z) oder

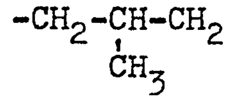

oder U bedeutet zusammen mit X eine Kette $=CH-CH_2-CH_2-$(E oder Z) oder U bedeutet zusammen mit Y eine Kette $-CH_2-CH_2-CH=$ (E oder Z), X bedeutet ein Wasserstoffatom oder zusammen mit U eine Kette $=CH-CH_2-CH_2-$(E oder Z) und Y bedeutet ein Wasserstoffatom oder mit U eine Kette $-CH_2-CH_2-CH=$ (E oder Z), dadurch gekennzeichnet,daß man ein Derivat der Glutaminsäure der Formel (II)

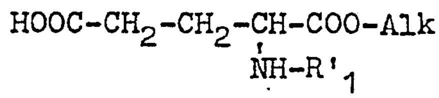 (II)

worin Alk einen $C_1$-$C_3$-Alkylrest bedeutet und $R'_1$ die für $R_1$ angegebene Bedeutung hat mit Ausnahme von Wasserstoff oder eine Schutzgruppe eines Amins bedeutet, mit einem Derivat der Formel (III), das in Form von getrennten Isomeren oder in Form einen Isomerengemisches vorliegen kann :

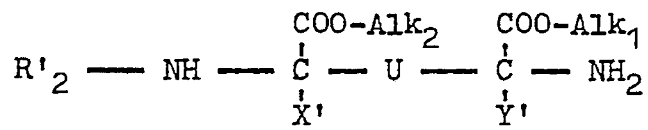 (III)

zur Reaktion bringt, worin U die angegebene Bedeutung hat, $Alk_1$ und $Alk_2$ einen $C_1$-$C_3$-Alkylrest darstellen, X′ und Y′ ein Wasserstoffatom, einen Rest $-COO-Alk_3$, wobei $Alk_3$ die Bedeutung von $Alk_1$ und $Alk_2$ hat, oder eine zusätzliche Bindung mit U bedeutet und $R'_2$ eine Schutzgruppe eines Amins oder eine Aminosäure oder ein Peptid aus 2, 3 oder 4 Aminosäuren bedeutet, deren Amin durch eine Schutzgruppe geschützt ist, um ein Produkt der Formel ($I_A$)

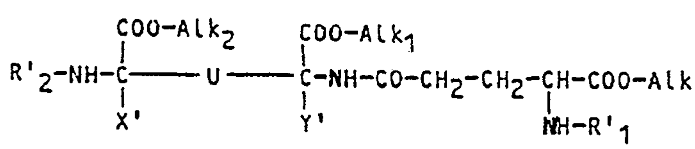 ($I_A$)

in allen seinen isomeren Formen oder in Form von Isomerengemischen zu erhalten, wobei $R'_1$, $R'_2$, Alk, $Alk_1$, $Alk_2$, X′, Y′ und U die angegebene Bedeutung haben, das man isoliert und gewünschtenfalls in ein Salz überführt oder es, falls notwendig und gewünscht, einer der folgenden Reaktionen in irgendeiner Reihenfolge unterwirft :

a) wenn X′ oder Y′ einen Rest $COO-Alk_3$ bedeuten : Decarboxylierung,

b) Schutzgruppenentfernung der Aminofunktionen,

c) Entalkylierung der Hydroxylfunktionen,

d) Amidierung der freien Aminofunktionen durch eine Aminosäure oder ein Peptid aus 2, 3 oder 4 Aminosäuren, deren Aminofunktion geschützt ist, dann Schutzgruppenentfernung dieser Aminofunktion,

e) Veresterung oder Salzbildung der Carboxylfunktionen durch eine Base,

f) Salzbildung der Aminofunktionen durch eine Säure.

2. Verfahren gemäß Anspruch 1, dadurch gekennzeichnet, daß man an dem Derivat der Formel ($I_A$) die folgenden Arbeitsgänge und in dieser Reihenfolge vornimmt :

– Entalkylierung der Carboxylfunktionen,

– Schutzgruppenentfernung der Aminofunktionen und Decarboxylierung,

– Amidierung oder Salzbildung der Aminofunktionen durch eine Säure und/oder Veresterung oder Salzbildung der Carboxylfunktionen durch eine Base.

3. Verfahren gemäß Anspruch 1 oder 2, dadurch gekennzeichnet, daß man als Ausgangsstoff ein Derivat der Glutaminsäure der Formel (II) mit D-Konfiguration verwendet.

4. Verfahren gemäß Anspruch 1, 2 oder 3, dadurch gekennzeichnet, daß man ein Produkt der Formel (I) herstellt, worin $R_3$ und $R_4$ einen Hydroxyrest bedeuten.

5. Verfahren gemäß einem der Ansprüche 1 bis 4, dadurch gekennzeichnet, daß man ein Produkt der For-

mel (I) herstellt, worin $R_5$ ein Wasserstoffatom bedeutet.

6. Verfahren gemäß einem der Ansprüche 1 bis 5, dadurch gekennzeichnet, daß man ein Produkt der Formel (I) herstellt, die namentlich folgendermaßen lauten :

2-Amino-6-(γ-D-glutamylamino)-4-methylenheptandisäure,
2-(L-Alanylamino)-6-(γ-D-glutamylamino)-4-methylenheptandisäure
sowie ihre Additionssalze.

**Patentansprüche für den Vertragsstaat : GR**

1. Verfahren zur Herstellung von Derivaten der Glutaminsäure sowie ihren Additionssalzen mit Mineral- oder organischen Säuren oder mit Basen unter allen isomeren Formen oder unter den Formen der Isomerengemische, dadurch gekennzeichnet, daß sie der allgemeinen Formel (I)

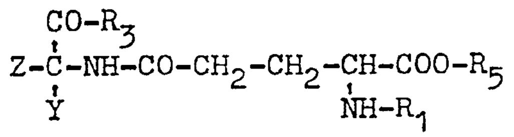
(I)

entsprechen, worin die Glutaminsäure die D- oder L-Konfiguration hat, $R_1$ ein Wasserstoffatom, eine Aminosäure oder ein Peptid, bestehend aus 2, 3 oder 4 Aminosäuren, oder eine Aminosäure oder ein Peptid, bestehend aus 2, 3 oder 4 Aminosäuren, deren Amin durch eine gesättigte oder ungesättigte, aliphatische $C_6$-$C_{24}$-Säure verestert ist, bedeutet oder $R_1$ bedeutet den Rest einer gesättigten oder ungesättigten, aliphatischen $C_6$-$C_{24}$-Säure, $R_5$ bedeutet ein Wasserstoffatom oder einen Alkylrest mit 1 bis 5 Kohlenstoffatomen, $R_3$ bedeutet einen Hydroxy-, $C_1$-$C_5$-Alkoxy-Rest oder eine Aminosäure, deren Amin durch einen $C_1$-$C_5$-Akylrest substituiert sein kann, Z bedeutet eine Gruppe der Formel

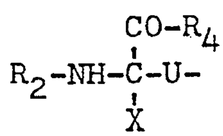

worin $R_2$ ein Wasserstoffatom, eine Aminosäure oder ein Peptid, bestehend aus 2, 3 oder 4 Aminosäuren, bedeutet, $R_4$ bedeutet einen Hydroxy-, $C_1$-$C_5$-Alkoxyrest oder eine Aminosäure, deren Amin durch einen $C_1$-$C_5$-Alkylrest substituiert sein kann, U bedeutet eine Kette

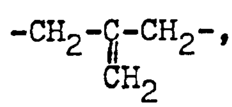

$-CH=CH-CH_2-$(E oder Z), $-CH_2-CH=CH-$(E oder Z) oder

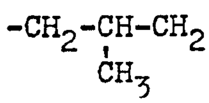

oder U bedeutet zusammen mit X eine Kette $=CH-CH_2-CH_2-$(E oder Z) oder U bedeutet zusammen mit Y eine Kette $-CH_2-CH_2-CH=$ (E oder Z), X bedeutet ein Wasserstoffatom oder zusammen mit U eine Kette $=CH-CH_2-CH_2-$(E oder Z) und Y bedeutet ein Wasserstoffatom oder mit U eine Kette $-CH_2-CH_2-CH=$ (E oder Z), dadurch gekennzeichnet, daß man ein Derivat der Glutaminsäure der Formel (II)

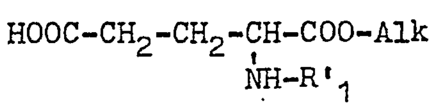
(II)

worin Alk einen $C_1$-$C_3$-Alkylrest bedeutet und $R'_1$ die für $R_1$ angegebene Bedeutung hat mit Ausnahme von Wasserstoff oder eine Schutzgruppe eines Amins bedeutet, mit einem Derivat der Formel (III), das in Form von getrennten Isomeren oder in Form von Isomerengemischen vorliegen kann :

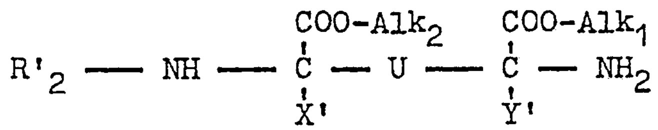

(III)

zur Reaktion bringt, worin U die angegebene Bedeutung hat, $Alk_1$ und $Alk_2$ einen $C_1$-$C_3$-Akylrest darstellen, X′ und Y′ ein Wasserstoffatom, einen Rest $-COO-Alk_3$, wobei $Alk_3$ die Bedeutung von $Alk_1$ und $Alk_2$ hat, oder eine zusätzliche Bindung mit U bedeutet und $R'_2$ eine Schutzgruppe eines Amins oder eine Aminosäure oder ein Peptid aus 2, 3 oder 4 Aminosäuren bedeutet, deren Amin durch eine Schutzgruppe geschützt ist, um ein Produkt der Formel ($I_A$)

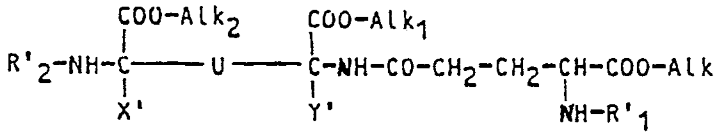

($I_A$)

in allen seinen isomeren Formen oder in Form von Isomerengemischen zu erhalten, wobei $R'_1$, $R'_2$, Alk, $Alk_1$, $Alk_2$, X′, Y′ und U die angegebene Bedeutung haben, daß man isoliert und gewünschtenfalls in ein Salz überführt oder es, falls notwendig und gewünscht, einer der folgenden Reaktionen in irgendeiner Reihenfolge unterwirft :

a) wenn X′ oder Y′ einen Rest COO-$Alk_3$ bedeuten : Decarboxylierung,
b) Schutzgruppenentfernung der Aminofunktionen,
c) Entalkylierung der Hydroxylfunktionen,
d) Amidierung der freien Aminofunktionen durch eine Aminosäure oder ein Peptid aus 2, 3 oder 4 Aminosäuren, deren Aminofunktion geschützt ist, dann Schutzgruppenentfernung dieser Aminofunktion,
e) Veresterung oder Salzbildung der Carboxylfunktionen durch eine Base,
f) Salzbildung der Aminofunktionen durch eine Säure.

2. Verfahren gemäß anspruch 1, dadurch gekennzeichnet, daß man an dem Derivat der Formel ($I_A$) die folgenden Arbeitsgänge und in dieser Reihenfoge vornimmt :

– Entalkylierung der Carboxylfunktionen,
– Schutzgruppenentfernung der Aminofunktionen und Decarboxylierung,
– Amidierung oder Salzbildung der Aminofunktionen durch eine Säure und/oder Veresterung oder Salzbildung der Carboxylfunktionen durch eine Base.

3. Verfahren gemäß Anspruch 1 oder 2, dadurch gekennzeichnet, daß man als Ausgangsstoff ein Derivat der Glutaminsäure der Formel (II) mit D-Konfiguration verwendet.

4. Verfahren gemäß Anspruch 1, 2 oder 3, dadurch gekennzeichnet, daß man ein Produkt der Formel (I) herstellt, worin $R_3$ und $R_4$ einen Hydroxyrest bedeuten.

5. Verfahren gemäß einem der Ansprüche 1 bis 4, dadurch gekennzeichnet, daß man ein Produkt der Formel (I) herstellt, worin $R_5$ ein Wasserstoffatom bedeutet.

6. Verfahren gemäß einem der Ansprüche 1 bis 5, dadurch gekennzeichnet, daß man ein Produkt der Formel (I) herstellt, die namentlich folgendermaßen lauten :

2-Amino-6-(γ-glutamylamino)-4-methylenheptandisäure,
2-(L-Alanylamino)-6-(γ-D-glutamylamino)-4-methylenheptandisäure
sowie ihre Additionssalze.

7. Verfahren zur Herstellung pharmazeutischer Zusammensetzungen, dadurch gekennzeichnet, daß man als aktives Prinzip mindestens eines der Produkte der Formel (I), wie in Anspruch 1 definiert, unter allen seinen isomeren Formen oder unter der Form von Isomerengemischen oder wenigstens eines seiner Additionssalze mit Mineral- oder organischen Säuren oder mit Basen in einer für diesen Vewendungszweck bestimmten Form einsetzt.

8. Verfahren zur Herstellung pharmazeutischer Zusammensetzungen, dadurch gekennzeichnet, daß man als aktives Prinzip wenigstens eines der Produkte der Formel (I), wie in Anspruch 6 definiert, unter allen seinen isomeren Formen oder unter der Form von Isomerengemischen oder wenigstens eines seiner Additionssalze

mit Mineral- oder organischen Säuren oder mit Basen in einer für diesen Verwendungsweck bestimmten Form einsetzt.